# EUROPEAN PATENT APPLICATION

(11) **EP 3 808 257 A1**
(43) Date of publication of application: **21.04.2021**
(21) Application number: 20212391.5
(22) Date of filing: 21.11.2012
(51) Int. Cl.: A61B 5/00, A61B 5/07, H04B 1/02, A61K 9/00, H04B 1/034

(54) **INGESTIBLE COMPOSITIONS COMPRISING A SHELF-LIFE STABILITY COMPONENT**

(30) Priority: 23.11.2011 US 201113304260
(62) Divisional of application: 12851774.5
(71) Applicant: Proteus Digital Health, Inc., Redwood City, CA 94065 (US)
(72) Inventor: HAFEZI, Hooman, Redwood City, California 94065-1233 (US); SCHMIDT, Raymond, San Mateo, California 94401 (US); CHING, Ai Ling, San Francisco, California 94122 (US)
(74) Representative: Murgitroyd & Company

(57) **Abstract**

Compositions that include a shelf-life stability component are provided. In some instances, the compositions are ingestible compositions which include the shelf-life stability component and an ingestible component. Aspects of the disclosure further include methods of making and using the compositions.

## Description

### CROSS REFERENCE TO RELATED APPLICATIONS

This application claims priority to U.S. Patent Application Serial No. 13/304,260 filed on November 23, 2011; the disclosure of which is herein incorporated by reference.

### INTRODUCTION

A variety of different ingestible compositions have been developed for nutritional, therapeutic and non-therapeutic uses. Examples of different types of ingestible compositions include orally ingestible tablets, capsules and liquids. A given orally ingestible formulation may include a variety of different components, such as active agents, carrier materials (including binders, bulking agents and other excipients), flavoring agents, coloring agents, etc. More recently, ingestible compositions which include a device component, such as an RFID tag or an ingestible event marker, have been developed.

As with many consumer products, ingestible compositions are not manufactured at the time of and location of use. Instead, they are generally manufactured at one or more fabrication facilities, stored for a period of time and then shipped to the end-user. Upon receipt, the end-user may further store them for a period of time before use.

During the multiple storage periods, and even manufacturing periods, such as mentioned above, the quality of the ingestible composition, e.g., in terms of effectiveness, may be degraded in some way. For example, exposure to humidity, elevated temperatures, microorganisms and oxidizing agents, as well other environmental hazards, can negatively impact the quality of the ingestible composition. Shelf-life stability of ingestible compositions is therefore a significant consideration in their manufacture and use.

### SUMMARY

Compositions that include a shelf-life stability component are provided. In some instances, the compositions are ingestible compositions which include the shelf-life stability component and an ingestible component. Aspects of the disclosure further include methods of making and using the compositions.

### BRIEF DESCRIPTION OF THE FIGURES

FIGS. 1A and 1B provide side and top views, respectively, of one aspect of an ingestible event marker (IEM);
FIG. 2 provides a side view of one aspect of an ingestible composition that includes a mono-layer protective barrier;
FIG. 3 provides a side view of one aspect of an ingestible composition that includes a protective barrier made of a homogeneous blend of two different materials;
FIG. 4 provides a side view of one aspect of an ingestible composition that includes a protective barrier made of a heterogeneous structure of two different materials;
FIGS. 5A and 5B provide side views of one aspect of an ingestible composition that includes a multi-layer protective barrier;
FIG. 6 provides a side view of one aspect of an ingestible composition that includes a protective barrier made of an inter-digitated structure of two different materials;
FIG. 7 provides a side view of one aspect of an ingestible composition that includes a protective barrier made of an overlapping structure of two different materials;
FIG. 8 provides a side view of one aspect of an ingestible composition that includes a multi-layer protective barrier;
FIG. 9 provides a side view of one aspect of an ingestible composition that includes a multi-layer protective barrier;
FIG. 10 provides a side view of one aspect of an ingestible composition that includes a galvanic protective barrier;
FIG. 11 provides a side view of one aspect of an ingestible composition that includes a mono-layer protective barrier with one or more fluid passageways;
FIG. 12 is a block diagram representation of one aspect of the event indicator system with dissimilar metals positioned on opposite ends;
FIG. 13 is a block diagram representation of another aspect of the event indicator system with dissimilar metals positioned on the same end and separated by a non-conducting material;
FIG. 14 shows ionic transfer or the current path through a conducting fluid when the event indicator system of FIG. 12 is in contact with conducting liquid and in an active state;
FIG. 14A shows an exploded view of the surface of dissimilar materials of FIG. 14;
FIG. 14B shows the event indicator system of FIG. 14 with a pH sensor unit;
FIG. 15 is a block diagram illustration of one aspect of the control device used in the system of FIGS. 12 and 13;
FIG. 16 is a functional block diagram of a demodulation circuit that performs coherent demodulation that may be present in a receiver, according to one aspect;
FIG. 17 illustrates a functional block diagram for a beacon module within a receiver, according to one aspect;
FIG. 18 is a block diagram of different functional modules that may be present in a receiver, according to one aspect;
FIG. 19 is a block diagram of a receiver, according to one aspect;
FIG. 20 provides a block diagram of a high frequency signal chain in a receiver, according to one aspect; and
FIG. 21 provides a diagram of how a system that includes a signal receiver and an ingestible event marker may be employed, according to one aspect.

### DETAILED DESCRIPTION

Compositions that include a shelf-life stability component are provided. In some instances, the compositions are ingestible compositions which include the shelf-life stability component and an ingestible component. Aspects of the disclosure further include methods of making and using the compositions.

### COMPOSITIONS

Aspects of the disclosure include compositions having shelf-life stability component physically associated with a minimally dimensioned component. A shelf-life stability component is a component that imparts shelf-life stability to the composition, in that the shelf-life stability component enhances the storage stability of the composition by a quantifiable measure as compared to a control composition that lacks the shelf-life stability component. Shelf-life stability components of interest may enhance the shelf-life stability of the composition as compared to a suitable control by a magnitude of two-fold or greater, such as five-fold or greater including ten-fold or greater, e.g., twenty-five-fold or greater. The presence of the shelf-life stability component allows the composition to be stable for extended periods of time during or following manufacture, where the ingestible composition may be stable for one year or longer, such as two years or longer, including five years or longer, following manufacture when the composition maintained under conditions in which the temperature ranges from 10 to 40°C, the pressure ranges from 0.5 to 2.0 ATM and the relative humidity ranges from 10 to 100%. By "stable" is meant that the functionality of the composition does not degrade to a point that the composition is no longer suitable for use in its intended purpose. For example, if the composition includes a circuitry component, e.g., an ingestible event marker (such as described in greater detail below) or a micro-battery, the circuitry component continues to function for its intended purpose for the period of time between manufacture and ingestion when stored under the conditions described above. If the composition includes an active pharmaceutical agent, the amount of active agent following the storage time period may be 85% or more, such as 90% or more, including 95% or more of the original amount present in the composition following manufacture, e.g., as determined using an HPLC protocol or other suitable analytical technique which can distinguish the amount of active agent from any degradation byproducts, such as oxidation byproducts.

Minimally dimensioned components may vary in dimension, and in some instances have a longest dimension of 30 mm or less, such as 20 mm or less, e.g., 10 mm or less. The volume of these minimally dimensioned components of interest may also vary, where the volume in some instances may be 25 mm³ or less, such as 15 mm³ or less, including 10 mm³ or less. Of interest as minimally dimensioned components are components that are susceptible at least partial degradation during storage. Such components may or may not include circuitry component, e.g., as described in greater detail below. Compositions of interest that may include a shelf-life stability component include ingestible compositions, micro-batteries, etc.

### INGESTIBLE COMPOSITIONS

Aspects of the disclosure include ingestible compositions. In these instances, ingestible compositions of interest include both an ingestible component and shelf-life stability component. As the compositions are ingestible, they are configured to be ingested or swallowed, e.g., taken into the stomach by drawing through the throat and esophagus with a voluntary muscular action. Accordingly, the compositions are dimensioned so as to be capable of being ingested. In some instances, the compositions have a longest dimension of 30 mm or less, such as 20 mm or less, e.g., 10 mm or less. The volume of the ingestible composition may also vary so long as the composition is suitable for ingestion, where the volume in some instances may be 25 mm³ or less, such as 15 mm³ or less, including 10 mm³ or less.

The ingestible component is a portion or part of the ingestible composition that is configured for ingestion. The ingestible component may vary widely and may include one or more subcomponents, e.g., a pharmaceutically acceptable solid carrier (which may or may not include an active agent), a device (which may or may not include electronic circuitry), etc.

In some instances, the ingestible component includes a pharmaceutically acceptable solid carrier. Pharmaceutically acceptable solid carrier configurations include tablet and capsule configurations. While the pharmaceutically acceptable solid carrier may have a solid configuration, the solid configuration may include a liquid component, such as is found in a liquid capsule, which includes a liquid component present in a solid capsule. In some instances, the pharmaceutically acceptable solid carrier is configured to impart a controlled release profile to an active agent that is associated with the pharmaceutically acceptable solid carrier. Examples of pharmaceutically acceptable solid carriers of interest can be found in Remington's Pharmaceutical Sciences, Mace Publishing Company, Philadelphia, Pa., 17th ed. (1985).

Where desired, the pharmaceutically acceptable solid carrier may include an active agent. Active agents of interest include pharmaceutically active agents as well as non-pharmaceutical active agents, such as diagnostic agents. The phrase "pharmaceutically active agent" (also referred to herein as drugs) refers to a compound or mixture of compounds which produces a physiological result, e.g., a beneficial or useful result, upon contact with a living organism, e.g., a mammal, such as a human. Pharmaceutically active agents are distinguishable from such components as excipients, carriers, diluents, lubricants, binders and other formulating aids, and encapsulating or otherwise protective components. The pharmaceutically active agent may be any molecule, as well as binding portion or fragment thereof, that is capable of modulating a biological process in a living subject. In certain aspects, the pharmaceutically active agent may be a substance used in the diagnosis, treatment, or prevention of a disease or as a component of a medication. The pharmaceutically active agent is capable of interacting with a target in a living subject. The target may be a number of different types of naturally occurring structures, where targets of interest include both intracellular and extracellular targets. Such targets may be proteins, phospholipids, nucleic acids and the like, where proteins are of particular interest. Specific proteinaceous targets of interest include, without limitation, enzymes, e.g., kinases, phosphatases, reductases, cyclooxygenases, proteases and the like, targets comprising domains involved in protein-protein interactions, such as the SH2, SH3, PTB and PDZ domains, structural proteins, e.g., actin, tubulin, etc., membrane receptors, immunoglobulins, e.g., IgE, cell adhesion receptors, such as integrins, etc., ion channels, transmembrane pumps, transcription factors, signaling proteins, and the like. Broad categories of active agents of interest include, but are not limited to: cardiovascular agents; pain-relief agents, e.g., analgesics, anesthetics, anti-inflammatory agents, etc.; nerve-acting agents; chemotherapeutic (e.g., anti-neoplastic) agents; neurological agents, e.g., anti-convulsants, etc. The amount of active agent that is present in the solid carrier may vary. In some instances, the amount of active agent that is present may range from 0.01 to 100% by weight.

Further examples of pharmaceutically acceptable solid carriers and active agents which may or may not be included therein are described in PCT application serial no. PCT/US2006/016370 published as WO/2006/116718; PCT application serial no. PCT/US2007/082563 published as WO/2008/052136; PCT application serial no. PCT/US2007/024225 published as WO/2008/063626; PCT application serial no. PCT/US2007/022257 published as WO/2008/066617; PCT application serial no. PCT/US2008/052845 published as WO/2008/095183; PCT application serial no. PCT/US2008/053999 published as WO/2008/101107; PCT application serial no. PCT/US2008/056296 published as WO/2008/112577; PCT application serial no. PCT/US2008/056299 published as WO/2008/112578; PCT application serial no. PCT/US2008/077753 published as WO2009/042812; PCT application serial no. PCT/US2008/085048 published as WO2009/070773; PCT application serial no. PCT/US2009/36231 published as WO2009/111664; PCT application serial no. PCT/US2009/049618 published as WO2010/005877; PCT application serial no. PCT/US2009/053721 published as WO2010/019778; PCT application serial no. PCT/US2009/060713 published as WO2010/045385; PCT application serial no. PCT/US2009/064472 published as WO2010/057049; PCT application serial no. PCT/US2009/067584 published as WO2010/068818; PCT application serial no. PCT/US2009/068128 published as WO2010/075115; PCT application serial no. PCT/US2010/020142 published as WO2010/080765; PCT application serial no. PCT/US2010/020140 published as WO2010/080764; PCT application serial no. PCT/US2010/020269 published as WO2010/080843; PCT application serial no. PCT/US2010/028518 published as WO2010/111403; PCT application serial no. PCT/US2010/032590 published as WO2010/129288; PCT application serial no. PCT/US2010/034186 published as WO2010/132331; PCT application serial no. PCT/US2010/055522 published as WO2011/057024; the disclosures of which are herein incorporated by reference.

In addition to or instead of a pharmaceutically acceptable solid carrier, ingestible compositions may include a device. The term "device" is used broadly to refer to a mechanical and/or electrical component configured for a particular purpose, where the device may or may not include a circuitry component.

Of interest as devices are ingestible devices, e.g., RFID-enabled devices; ingestible event markers, etc. An ingestible event marker (IEM) is a device that is dimensioned to be ingestible and includes an identifier circuitry component and, optionally, a current path extender, e.g., a membrane, sometimes referred to herein as a "skirt." To illustrate, various aspects of an IEM may include a control device for altering conductance; and a partial power source. The partial power source may include a first material electrically coupled to the control device; and a second material electrically coupled to the control device and electrically isolated from the first material.

Upon ingestion, the IEM contacts a conducting fluid, e.g., stomach fluid. When the IEM is in contact with the conducting liquid, a current path is formed through the conducting liquid between the first and second materials. The voltage potential created between the materials provides the power for operating the IEM as well as produces the current flow through the conducting fluid and the system. In one aspect, the IEM operates in direct current mode. In an alternative aspect, the IEM controls the direction of the current so that the direction of current is reversed in a cyclic manner, similar to alternating current. The current path through the system is controlled by the control device. Completion of the current path allows for the current to flow and in turn a receiver can detect the presence of the current and recognize that the system has been activated and the desired event is occurring or has occurred.

In one aspect, the two materials are similar in function to the two electrodes needed for a direct current power source, such as a battery. The conducting liquid acts as the electrolyte needed to complete the power source. The completed power source described is defined by the electrochemical reaction between the materials of the IEM and enabled by the fluids of the body. The completed power source may be viewed as a power source that exploits electrochemical conduction in an ionic or a conducting solution such as gastric fluid, blood, or other bodily fluids and some tissues.

In certain aspects, the complete power source or supply is one that is made up of active electrode materials, electrolytes, and inactive materials, such as current collectors, packaging, etc. The active materials are any pair of materials with different electrochemical potentials. Suitable materials are not restricted to metals, and in certain aspects the paired materials are chosen from metals and non-metals, e.g., a pair made up of a metal (such as Mg) and a salt (such as Cul). With respect to the active electrode materials, any pairing of substances - metals, salts, or intercalation compounds - with suitably different electrochemical potentials (voltage) and low interfacial resistance are suitable. Where desired, the voltage provided by the two dissimilar electrochemical materials upon contact of the materials of the power source with the target physiological site is 0.001 V or higher, including 0.01 V or higher, such as 0.1 V or higher, e.g., 0.3 V or higher, including 0.5 volts or higher, and including 1.0 volts or higher, where in certain aspects, the voltage ranges from about 0.001 to about 10 volts, such as from about 0.01 to about 10 V.

Anode materials of interest include, but are not limited to: magnesium, zinc, sodium, lithium, iron and alloys thereof, e.g., Al and Zn alloys of Mg, which may or may not be intercalated with a variety of materials such, as graphite with Li, K, Ca, Na, Mg, and the like. Cathode materials of interest include, but are not limited to, copper salts, such as copper salts of iodide, chloride, bromide, sulfate, formate, Fe³⁺ salts, e.g., orthophosphate, pyrophosphate, etc. One or both of the metals may be doped with a non-metal, for example to enhance the voltage output of the battery. Non-metals that may be used as doping agents in certain aspects include, but are not limited to: sulfur, iodine and the like. In certain aspects, the electrode materials are cuprous iodine (Cul) or cuprous chloride (CuCI) as the anode and magnesium (Mg) metal or magnesium alloy as the cathode. Aspects of the present disclosure use electrode materials that are not harmful to the human body.

With respect to current signatures, the current signatures may distinguish one class of ingestible event marker from other types or may be universally unique, such as where the current signature is analogous to a human fingerprint which is distinct from any other fingerprint of any other individual and therefore uniquely identifies an individual on a universal level. In various aspects, the control circuit may generate a variety of different types of communications, including but not limited to: RF signals, magnetic signals, conductive (near-field) signals, acoustic signals, etc.

In various aspects, the IEM may further comprise a current path extender such as a membrane which, for example, produces a virtual dipole length between the pair of transmission elements that is larger than the actual dipole length. In addition to controlling the magnitude of the current path between the materials, a membrane (sometimes referred to herein as "amplifier" or "skirt") is used to increase the "length" of the current path and, hence, act to boost the conductance path, as disclosed in the U.S. Patent Application Publication No. US 2009-0082645 A1 entitled, "In-Body Device with Virtual Dipole Signal Amplification" published March 26, 2009, and in the U.S. Patent No. 7,978,064 entitled, "Communication System with Partial Power Source" dated July 12, 2011 the entire content of which are incorporated herein by reference.

Receivers, sometimes referred to herein as a "detector" may detect the communication, e.g., current. Receivers may not require any additional cable or hard wire connection between the device and a receiver of the communication, sometimes referred to herein as a detector.

In the ingestible composition of interest, the IEM may be stably associated in some manner to another ingestible component, e.g., pharmaceutically acceptable carrier component (e.g., as described above). By "stably associated" is meant that the IEM and second ingestible component, e.g., a pharmaceutically acceptable carrier component, do not separate from each other, at least until administered to the subject in need thereof, e.g., by ingestion. As the lEMs are dimensioned to be ingestible, they are sized so that they can be placed in a mammalian, e.g., human or animal, mouth and swallowed. In some instances, IEMs of the disclosure have a longest dimension that is 30 mm or less, such as 20 mm or less, including 5 mm or less.

Various aspects of ingestible event markers of interest (including protocols for the fabrication thereof) are described in PCT application serial no. PCT/US2006/016370 published as WO/2006/116718; PCT application serial no. PCT/US2007/082563 published as WO/2008/052136; PCT application serial no. PCT/US2007/024225 published as WO/2008/063626; PCT application serial no. PCT/US2007/022257 published as WO/2008/066617; PCT application serial no. PCT/US2008/052845 published as WO/2008/095183; PCT application serial no. PCT/US2008/053999 published as WO/2008/101107; PCT application serial no. PCT/US2008/056296 published as WO/2008/112577; PCT application serial no. PCT/US2008/056299 published as WO/2008/112578; PCT application serial no. PCT/US2008/077753 published as WO2009/042812; PCT application serial no. PCT/US2008/085048 published as WO2009/070773; PCT application serial no. PCT/US2009/36231 published as WO2009/111664; PCT application serial no. PCT/US2009/049618 published as WO2010/005877; PCT application serial no. PCT/US2009/053721 published as WO2010/019778; PCT application serial no. PCT/US2009/060713 published as WO2010/045385; PCT application serial no. PCT/US2009/064472 published as WO2010/057049; PCT application serial no. PCT/US2009/067584 published as WO2010/068818; PCT application serial no. PCT/US2009/068128 published as WO2010/075115; PCT application serial no. PCT/US2010/020142 published as WO2010/080765; PCT application serial no. PCT/US2010/020140 published as WO2010/080764; PCT application serial no. PCT/US2010/020269 published as WO2010/080843; PCT application serial no. PCT/US2010/028518 published as WO2010/111403; PCT application serial no. PCT/US2010/032590 published as WO2010/129288; PCT application serial no. PCT/US2010/034186 published as WO2010/132331; PCT application serial no. PCT/US2010/055522 published as WO2011/057024; the disclosures of which are herein incorporated by reference.

In certain aspects, the ingestible event markers are disrupted upon administration to a subject. As such, in certain aspects, the compositions are physically broken, e.g., dissolved, degraded, eroded, etc., following delivery to a body, e.g., via ingestion, injection, etc. The compositions of these aspects are distinguished from devices that are configured to be ingested and survive transit through the gastrointestinal tract substantially, if not completely, intact.

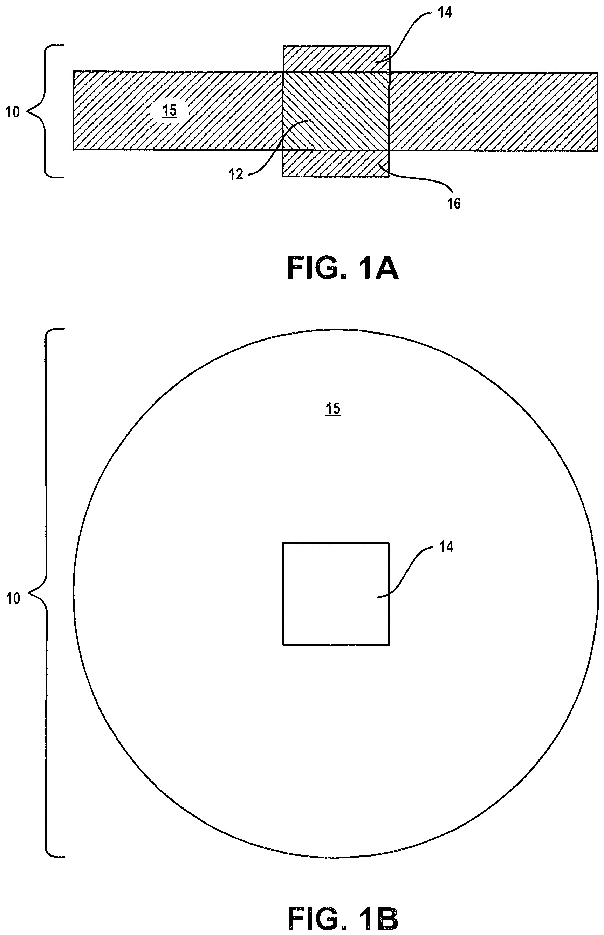

FIG. 1A provides a view of an aspect of an IEM of interest which has a current extender in the form of a membrane that extends beyond the outer edges of the signal transmission elements to provide a virtual dipole having a length that is longer than the actual dipole between the signal transmission elements. As shown in FIG. 1A, the IEM 10 includes integrated circuit 12, having a first electrochemical material 14 (which may comprise two distinct material layers) and a second electrochemical material 16. Also shown is disc shaped membrane 15. FIG. 1B provides an overhead view of the IEM shown in FIG. 1A, showing the disc shape of first electrochemical material 14 and the positioning of the first electrochemical material in the center of disc shaped membrane 15. The distance that the edge of the membrane may extend beyond the edge of electrodes may vary, and in certain aspects is 0.05 mm or more, e.g., 0.1 mm or more, including 1.0 mm or more, such as 5.0 mm or more and including 10 mm or more, where the distance may not exceed 100 mm in certain aspects.

As can be seen in the aspect depicted in FIGS. 1A to 1B, the first and second electrochemical materials may have any convenient shape, e.g., square, disc, etc. The disc shaped membrane 15 is a planar disc structure, where the edge of the membrane extends beyond the edge of the first and second electrochemical materials. In the depicted aspect, the radius of the membrane is longer than the radius of the first and second electrochemical materials, e.g., by 1mm or more, such as by 10 mm or more.

Membranes may have "two-dimensional" or "three-dimensional" configurations, as desired. Membrane configurations of interest are further described in PCT application serial no. US2008/077753 published as WO2009/042812, PCT application serial no. US2010/020142 published as WO2010/080765 as well as PCT application serial no. US2010/032590 published as WO2010/129288; the disclosures of which are herein incorporated by reference.

The membrane may be fabricated from a number of different materials, where the membrane may be made of a single material or be a composite of two or more different types of materials, as developed in greater detail below. In certain instances, the membrane may have a mechanical strength sufficient to withstand the mechanical forces typical of the gastrointestinal (GI) tract without folding onto itself and losing its shape. This desired mechanical strength may be chosen to last for at least the duration of the communication, which may be 1 second or longer, such as at least 1 minute or longer, up to 6 hours or longer. In certain aspects, the desired mechanical strength is selected to last at least for a period of time ranging from 1 to 30 minutes. The desired mechanical strength can be achieved by proper selection of polymer and/or fillers, or mechanical design (e.g., lamination of multiple layers, or curvature of the amplifier surface) to increase the mechanical strength of the final structure.

Membranes of the disclosure are ones that are electrically insulating. As such, the materials from which the membranes are fabricated are electrically insulating materials. A given material is electrically insulating if it has a resistivity that is two times or greater than the medium in which the device operates, e.g., stomach fluid, such as ten times or greater, including 100 times or greater than the medium in which the device operates.

Where desired, an active agent (e.g., as described above) may be present in one or more of the IEM components, e.g., in the electrochemical materials, the support, the membrane, etc. Examples of such configurations are described in PCT application serial no. US2010/032590 published as WO2010/129288; the disclosures of which are herein incorporated by reference.

### Other Minimally Dimensioned Components

Aspects of the disclosure further include compositions that are not necessarily ingestible. As summarized above, such compositions may include a shelf-life stability components (e.g., as summarized above and described in greater detail below) physically associated with a minimally dimensioned component. While the minimally dimensioned component may vary, e.g., as described above, in some instances the minimally dimensioned component is a micro-battery. Micro-batteries of interest may include "all-solid" batteries, and may include components of a battery, such as current collectors, positive and negative electrodes, an electrolyte, in a minimally dimensioned structure, e.g., as described above. In some instances, micro-batteries of interest are thin films, which may be obtained by deposition, such as by physical vapor deposition (PVD) or chemical vapor deposition (CVD). The micro-battery may take a variety of different configurations, such as but not limited to: a chip configuration, a cylinder configuration, a spherical configuration, a disc configuration, etc., where a particular configuration may be selected based on intended application, method of manufacture, etc. In certain embodiments, the micro-battery is dimensioned to have a width ranging from about 0.05 mm to about 1 mm, such as from about 0.1 mm to about 0.2 mm; a length ranging from about 0.05 mm to about 1 mm, such as from about 0.1 mm to about 0.2 mm and a height ranging from about 0.1 mm to about 1 mm, such as from about 0.05 mm to about 0.3 mm, including from about 0.1 mm to about 0.2 mm. In certain embodiments the micro-battery is 1 mm³ or smaller, such as 0.1 mm³ or smaller, including 0.2 mm³ or smaller.

### Shelf-Life Stability Component

As summarized above, an aspect of compositions of interest is a shelf-life stability component. Shelf-life stability components are elements of the compositions that enhance shelf-life stability of the composition as compared to a suitable control, e.g., as described above. The shelf-life stability components may vary widely, and may or may not be integrated with one or more other components of the compositions, e.g., a pharmaceutically acceptable solid carrier, an ingestible event marker, a micro-battery, etc. Furthermore, a given composition may include a single shelf-life stability component or two or more distinct shelf-life stability components, as desired. Examples of different types of the shelf-life stability components of interest include, but are not limited to: a water vapor desensitizer (e.g., a protective barrier, a desiccant, etc.), an electrochemical material variant that imparts shelf-life stability, an antioxidant, a stabilizer, or combination thereof, etc.

Of interest as shelf-life stability components are water vapor desensitizers. Water vapor desensitizers are components that reduce the sensitivity of the ingestible component or portions thereof to the deleterious effects of water vapor which may be present in the environment of the ingestible composition. The deleterious effects are harmful results of exposure to the water vapor, where examples of such effects include loss or chemical change of material, color change, loss of performance, etc. The magnitude of the deleterious effect reduction may vary, and may be 5% or greater, such as 10% or greater, including 25% or greater. The particular protocol for determining such magnitude may vary depending on the particular deleterious effect of interest. The water vapor desensitizers of interest include, but are not limited to: protective barriers, water vapor sequestering agents, etc.

In some instances, the water vapor desensitizer is a protective barrier. Protective barriers of interest include any structure or element that functions as an obstruction, hindrance, or impediment to the passage of water vapor from one portion of the ingestible composition to another, e.g., from the exterior of the ingestible composition to another region of the ingestible composition, e.g., an interior location that houses the IEM 10. Of interest as the protective barriers are those barriers that rapidly disrupt upon contact with a liquid, such as an aqueous liquid, e.g., stomach acid. By "rapidly disrupt" is meant that, upon contact with the liquid, the barrier is compromised in some fashion, such that it ceases to function as a complete barrier in a limited period of time, e.g., 60 minutes or less, such as 15 minutes or less, including 2 minutes or less. The protective barrier may be disrupted according to a number of different mechanisms, such as physical disruption, dissolution, etc.

The protective barriers may enclose an entire ingestible composition or a component thereof (e.g., the IEM 10) or be present on just a portion (e.g., one or more surfaces) of an ingestible composition or component thereof, as desired. The dimensions of a given barrier may vary, and in some instances the barrier has a thickness of 10 µm or greater, such as 25 µm or greater, including 50 µm or greater. In some instances, the thickness ranges from 10 to 1000 µm, such as 25 to 500 µm including 50 to 200 µm. The protective barriers may have a variety of different configurations, ranging from homogenous layers of a single material to heterogeneous layers of two or more materials to multilayer structures of two or more materials. Examples of various types of the protective barriers of interest are now described in greater detail.

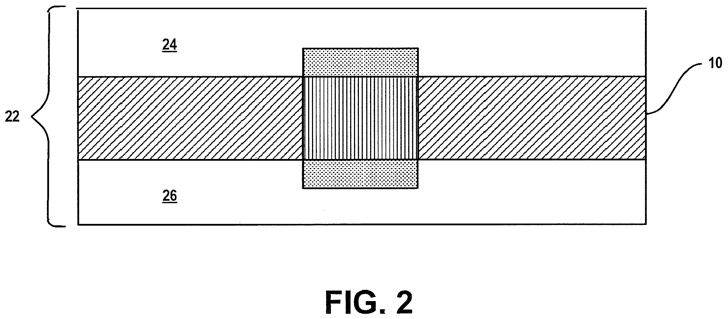

FIG. 2 provides a side view of an ingestible composition which includes a mono-layer protective barrier made of a single material and the IEM device 10. In FIG. 2, ingestible composition 22 includes the IEM component 10, e.g., as described in FIGS. 1A and 1B, and first and second protective barriers, 24 and 26, present on opposing sides of the IEM 10 and each in the form of a single homogenous layer. The thickness of each protective barrier may vary, where in some instances the thickness ranges from 25 to 500 µm including 50 to 200 µm. Each protective barrier may include a single material, or be a homogeneous mixture of two or more different materials, as reviewed in greater detail below.

A variety of different materials may be employed in the protective barriers (e.g., the protective layers 24 and 26), where materials of interest are those that impart hydrophobicity to the layer such that the layer acts as a suitable water vapor desensitizer. In addition to acting as a water vapor barrier prior to contact with a liquid, the protective barrier may also be made up of a material that imparts the desired rapid disruptability to the protective barrier upon contact of with a liquid.

Materials of interest include, but are not limited to, lipids and functionally analogous materials which are solid at room temperature, are suitable for ingestion, are non-toxic and dissociate from each other (e.g., melt or dissolve) at internal body temperatures (e.g., core body temperatures, where such materials may be referred to as low-melting point materials). Lipids of interest include fatty acyls, glycerolipids, glycerophospholipids, etc. Lipid materials that find use in protective barriers include, but are not limited to: long chain organic materials, e.g., waxes, such as acrawax, bayberry wax, beeswax, candelilla wax, castor wax, carnauba wax, ceresin wax, coconut oil, cotton seed oil, esparto wax, glycowax, jojoba wax, Japan wax, lignite wax, linear polyethylene wax, microcrystalline petroleum wax, montan wax, olive oil, ouricouri wax, ozokerite wax, paraffin wax, rice bran wax, shellac wax, silicone waxes, synthetic waxes, sugarcane wax, cetyl palmitate, etc.; fatty alcohols, e.g., cetyl alcohol, lanolin alcohol, stearyl alcohol, etc.; fatty acids, such as lauric acid, myristic acid, palmitic acid, stearic acid, behenic acid, lignoceric acid, ceratic acid, montanoic acid, isostearic acid, isononanoic acid, 2-ethylhexanoic acid, oleic acid, ricinoleic acid, linoleic acid, linolenic acid, erucic acid, soybean fatty acid, linseed fatty acid, dehydrated castor fatty acid, tall oil fatty acid, tung oil fatty acid, sunflower fatty acid, safflower fatty acid, etc.; phospholipids; and triglycerides, etc.

The protective barriers of interest may further include pharmaceutically acceptable polymeric materials, including but not limited to, cellulosic materials, such as ethyl cellulose, cellulose acetate phthalate, cellulose acetate trimaletate, hydroxy propyl methylcellulose phthalate, polyvinyl acetate phthalate, polyvinyl alcohol phthalate, shellac; hydrogels and gel-forming materials, such as carboxyvinyl polymers, sodium alginate, sodium carmellose, calcium carmellose, sodium carboxymethyl starch, poly vinyl alcohol, hydroxyethyl cellulose, methyl cellulose, ethyl cellulose, gelatin, starch, and cellulose based cross-linked polymers in which the degree of crosslinking is low so as to facilitate adsorption of water and expansion of the polymer matrix, hydroxypropyl cellulose, hydroxypropyl methylcellulose, polyvinylpyrrolidone, crosslinked starch, microcrystalline cellulose, chitin, pullulan, collagen, casein, agar, gum arabic, sodium carboxymethyl cellulose, (swellable hydrophilic polymers) poly(hydroxyalkyl methacrylate) (molecular weight 5 k to 5000 k), polyvinylpyrrolidone (molecular weight 10 k to 360 k), anionic and cationic hydrogels, zein, polyvinyl alcohol having a low acetate residual, a swellable mixture of agar and carboxymethyl cellulose, copolymers of maleic anhydride and styrene, ethylene, propylene or isobutylene, pectin (molecular weight 30 k to 300 k), polysaccharides such as agar, acacia, karaya, tragacanth, algins and guar, polyethylene oxides (molecular weight 100 k to 5000 k), diesters of polyglucan, crosslinked polyvinyl alcohol and poly N-vinyl-2-pyrrolidone, hydrophilic polymers such as polysaccharides, methyl cellulose, sodium or calcium carboxymethyl cellulose, hydroxypropyl methyl cellulose, hydroxypropyl cellulose, hydroxyethyl cellulose, nitro cellulose, carboxymethyl cellulose, cellulose ethers, methyl ethyl cellulose, ethylhydroxy ethylcellulose, cellulose acetate, cellulose butyrate, cellulose propionate, gelatin, starch, maltodextrin, pullulan, polyvinyl pyrrolidone, polyvinyl alcohol, polyvinyl acetate, glycerol fatty acid esters, natural gums, lecithins, pectin, alginates, ammonia alginate, sodium, calcium, potassium alginates, propylene glycol alginate, agar, and gums such as arabic, karaya, locust bean, tragacanth, carrageens, guar, xanthan, scleroglucan and mixtures and blends thereof, pharmaceutically acceptable acrylic polymers, including but not limited to acrylic acid and methacrylic acid copolymers, methyl methacrylate copolymers, ethoxyethyl methacrylates, cyanoethyl methacrylate, poly(acrylic acid), poly(methacrylic acid), methacrylic acid alkylamide copolymer, poly(methyl methacrylate), polymethacrylate, poly(methyl methacrylate) copolymer, polyacrylamide, aminoalkyl methacrylate copolymer, poly(methacrylic acid anhydride), and glycidyl methacrylate copolymers, etc.;

Also of interest as materials for protective barriers are ingestible metallic materials, e.g., gold, silver, titanium, copper, iron, magnesium, etc, as well as combinations thereof (see e.g., the galvanic protective layers described in greater detail below). Also of interest as materials for protective barriers are carbon allotropes having the desired properties, such as graphite, amorphous carbon, etc.

While the protective layer may be made up of a single type of material, in some instances the protective layer may be a homogenous blend (e.g., uniform mixture) of two or more different materials, where the second material may or may not be a material such as listed above, or another type of material which desirably modifies the properties of the first material. By homogeneous blend is meant a uniform mixture of the two or more materials. Accordingly, the protective barrier may not include regions or domains of a substantial volume that include only one type of material to the exclusion of the other. When present, the weight ratio of first to second material may vary, and in some instances may range from 1% to 99%, such as 25% to 75% and including 25% to 35%.

In some instances, the second material may enhance disruptability of the layer upon contact with a liquid, as desired, where the enhancement by the particular mechanism on the disruptability may vary. For example, the second material may be a solubilizing agent that enhances solubility of the layer, such that the two or more distinct materials making up the protective barrier include a first material and a second material that solubilizes the first material. Solubilizing agents of interest include, but are not limited to, emulsifiers (e.g., surfactants), enzymes, pH sensitive materials, etc. Surfactants of interest include pharmaceutically acceptable anionic surfactants, cationic surfactants, amphoteric (amphipathic/amphiphilic) surfactants, and non-ionic surfactants. Suitable pharmaceutically acceptable anionic surfactants include, for example, monovalent alkyl carboxylates, acyl lactylates, alkyl ether carboxylates, N-acyl sarcosinates, polyvalent alkyl carbonates, N-acyl glutamates, fatty acid-polypeptide condensates, sulfuric acid esters, and alkyl sulfates. Suitable pharmaceutically acceptable non-ionic surfactants include, for example, polyoxyethylene compounds, lecithin, ethoxylated alcohols, ethoxylated esters, ethoxylated amides, polyoxypropylene compounds, propoxylate alcohols, ethoxylated/propoxylated block polymers, and propoxylated esters, alkanolamides, amine oxides, fatty acid esters of polyhydric alcohols, ethylene glycol esters, diethylene glycol esters, propylene glycol esters, glyceryl esters, polyglyceryl fatty acid esters, SPAN's (e.g., sorbitan esters), TWEEN's sucrose esters, and glucose (dextrose) esters. Other suitable pharmaceutically acceptable surfactants/co-solvents (solubilizing) agents include acacia, benzalkonium chloride, cholesterol, emulsifying wax, docusate sodium, glyceryl monostearate, lanolin alcohols, lecithin, poloxamer, poloxytheylene castor oil derivatives, poloxyethylene sorbitan fatty acid esters, poloxyethylene stearates, sodium lauryl sulfates, sorbitan esters, stearic acid, and triethanolamine. Mixed surfactant/wetting agent systems are also useful in conjunction with the present disclosure. Examples of such mixed systems include, for example, sodium lauryl sulfate/polyethylene glycol (PEG) 6000 and sodium lauryl sulfate/PEG 6000/stearic acid. Enzymes may also find use as solubilizers, such as where the first material is a substrate for the enzyme. Examples of enzymes of interest include, but are not limited to hydrolases, e.g., esterases; oxidoreductases, etc. Also of interest are pH sensitive materials, in which the material is insoluble / impenetrable during storage, but soluble at low pH, e.g., a pH less than 6, such as a pH less than 5. Examples of such materials include, but are not limited to: methacrylate and methacrylic acids, such as EPO (cationic copolymer based on dimethylaminoethyl methacrylate, butyl methacrylate, and methyl methacrylate), etc. Also of interest as solubilizing materials are materials that generate heat upon contact with an aqueous solution, such as stomach fluid, e.g., where such materials may increase the rate at which the protective material melts. Examples of such materials include, but are not limited to: salts with high enthalpy of solution, e.g., magnesium sulfate, calcium chloride, etc.

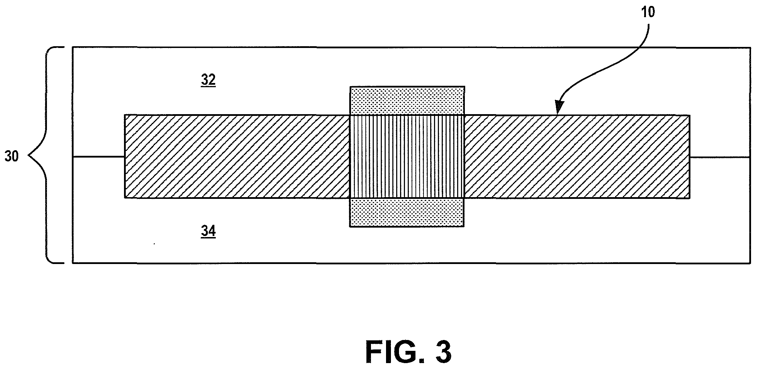

One type of the protective barrier of interest that includes two or more different materials is a protective barrier that is made up of a pharmaceutical tablet carrier material and a barrier material, e.g., as illustrated in FIG. 3. In FIG. 3, ingestible composition 30 includes the IEM device 10 which is sandwiched between first and second tablet halves 32 and 34 such that the ingestible composition 30 is in the form of a tablet. Each tablet half 32 and 34 includes a fused blend of a first tablet carrier material and a second protective barrier material. In the configuration shown in FIG. 3, the protective barrier material is present throughout each tablet half 32 and 34. An alternative configuration of interest is one which only an outer coating of surrounding the ingestible composition is made up of the blend of a carrier material and a second protective barrier material. In such instances, the coating may be any convenient thickness, e.g., 100 µ or thinner, such as 10 µ or thinner, including 1 µ or thinner.

The first tablet carrier material is made of one or more pharmaceutically acceptable tablet excipient materials. Tablet carrier materials of interest include, but are not limited to: fillers or extenders, such as starches, lactose, sucrose, glucose, mannitol, and/or silicic acid, talc; binders such as carboxymethylcellulose, ethyl cellulose and cellulose acetate, alginates, gelatin, polyvinyl pyrrolidone, sucrose and/or acacia; humectants such as glycerol; disintegrating agents such as agar-agar, calcium carbonate, potato or tapioca starch, alginic acid, silicates, and/or sodium carbonate; solution retarding agents such as paraffin; absorption accelerators such as quaternary ammonium compounds; wetting agents such as cetyl alcohol and/or glycerol monostearate; absorbents such as kaolin and/or bentonite clay; lubricants such as talc, calcium stearate, magnesium stearate, solid polyethylene glycols, sodium lauryl sulfate, and/or mixtures thereof; coloring agents; and buffering agents. Antioxidants can also be present in the pharmaceutical compositions of the disclosure. Examples of pharmaceutically acceptable antioxidants include: water-soluble antioxidants such as ascorbic acid, cysteine hydrochloride, sodium bisulfate, sodium metabisulfate sodium sulfite and the like; oil-soluble antioxidants such as ascorbyl palmitate, butylated hydroxyanisole (BHA), butylated hydroxytoluene (BHT), lecithin, propyl gallate, alpha-tocopherol, and the like; and metal-chelating agents such as citric acid, ethylenediamine tetraacetic acid (EDTA), sorbitol, tartaric acid, phosphoric acid, and the like.

The second protective barrier material may be a material made up of one or more ingredients, where the material melts at an elevated temperature in a manner that causes the second material to fill void spaces, e.g., pores, in the first carrier component. The elevated temperature at which the second material melts is one at which the first material does not physically change and one at which the components of the ingestible composition, e.g., the IEM device, are not damaged. In some instances, the elevated temperature at which the protective barrier material melts ranges from 25°C to 160°C, such as 80°C to 120°C. Any convenient material may be selected as the second material, where materials of interest include, but are not limited to: lipid materials (e.g., as described above), waxes, oils, and the like.

Ingestible compositions as shown in FIG. 3 may be produced using any convenient protocol. In some instances, a variation of the IEM tablet production protocols disclosed in PCT Application Serial No. PCT/US2006/016370 published as WO2006/116718; PCT Application Serial No. PCT/ US2010/020142 published as WO2010/080765 and PCT Application Serial No. PCT/ US2010/034186 published as 2010/132331 (the disclosures of which applications are incorporated by reference) is employed. In the variation that is employed, the tablet precursor material is a blend of a tablet carrier material and protective material, where examples of these types of materials are provided above. The weight ratio of tablet carrier material to protective barrier material in this precursor blend may vary. In some instances, the weight ratio of these two types of materials in the precursor blends ranges from 0.5 to 80%, such as 1 to 50%, e.g., 10 to 40% protective material. During fabrication, following tablet pressing the resultant composition may be heated to a sufficient temperature to fuse the protective material of the tablet carrier and thereby seal the pores of the tablet. While the temperature to which the tablet is elevated during this fusing step may vary, in some instances this fusing temperature ranges from 25°C to 160°C, such as 80°C to 120°C. The duration at which the tablet is held at this fusing temperature is one sufficient for the protective material to melt and fill pores present in the tablet structure, and in some instances ranges from 0.1 to 4 hours, such as 5 to 60 min, including 10 to 30 min.

Another type of the protective barrier of interest that includes two or more different materials is a barrier that is made up of a first protective barrier material component and a second solubilizer material of the first protective barrier component material. For example, the protective barrier material may be a lipid material, e.g., as described above. The second solubilizer material may be a component that enhances solubility of the lipid material upon contact with an aqueous medium, where examples of such lipid solubilizing materials include surfactants, e.g., as described above. The weight ratio of lipid material to solubilizer material may vary. In some instances, the weight ratio of these two types of materials ranges from 0.5% to 80%, such as 5% to 60% protective barrier material.

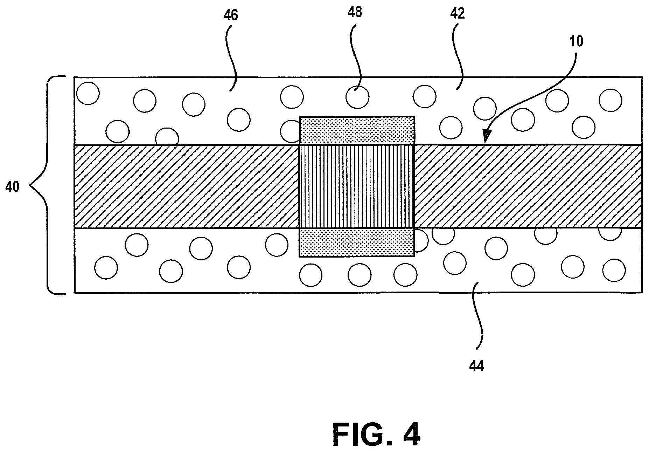

Instead of homogenous blend of two or more different materials, the protective layer may be a heterogeneous structure of two or more different materials, where regions (e.g., domains) of a second material, such as a water soluble material (e.g., a hydrogel, salt, etc.), are interspersed in regions of a hydrophobic material, e.g., a lipid material. FIG. 4 provides an illustration of such an ingestible composition. In FIG. 4, an ingestible composition 40 includes the IEM 10 position between two protective barriers, 42 and 44. Each of protective barriers 42 and 44 includes a first protective barrier material 46, e.g., as described above, and second regions or domains of a solubilizing material 48, e.g., as described above.

Protective barriers finding use as shelf-life stability components also include multilayer structures made up of two or more different materials. Of interest are multilayer structures of two or more materials, where the two materials may have differential properties that promote disruption of the protective barrier upon contact with a liquid. For example, the two or more distinct materials may exhibit different aqueous medium solubilities, such as one of the materials is more soluble in an aqueous medium than the other material. Alternatively, the two or more distinct materials may exhibit different aqueous medium physical properties, e.g., where one of the materials expands or shrinks in a manner different from the other upon contact with an aqueous medium, or where one of the materials produces gas upon contact with an aqueous medium, where the gas disrupts the barrier.

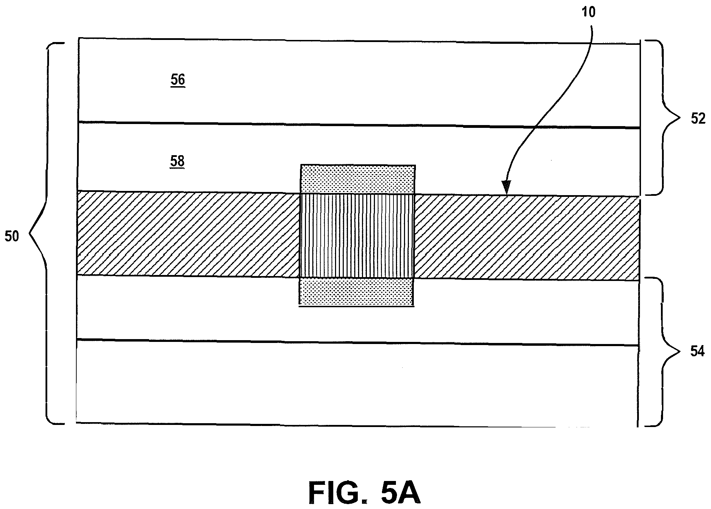

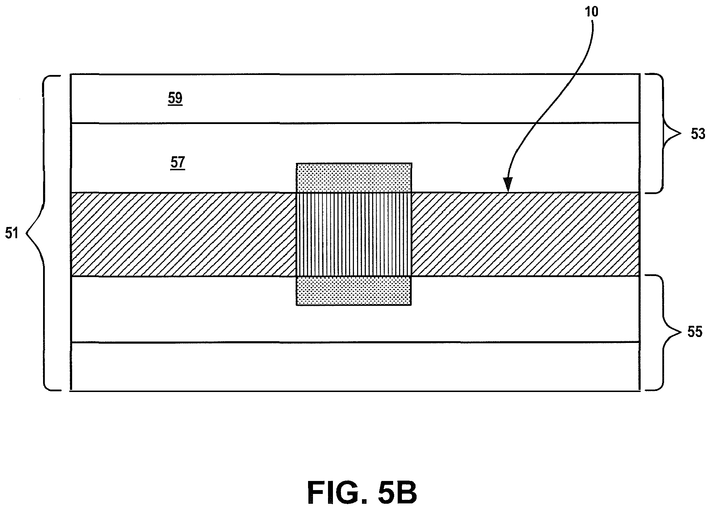

In one example of a multilayer protective barrier of interest, the multilayer structure is made up of a first layer of a protective barrier material and a second layer of a disrupting material that has a greater solubility in an aqueous medium than the first layer. An example of such a multilayer protective barrier is shown in the ingestible composition depicted in FIG. 5A. In FIG. 5A, an ingestible composition 50 includes the IEM device 10 sandwiched between first and second multilayer protective barriers 52 and 54. Each multilayer protective barrier 52 and 54 is made up of a first layer 56 of a protective material, e.g., as described above, and a second layer 58 of a disrupting material that is more soluble in an aqueous medium that the protective material. Examples of disrupting materials that may make the second layer in such configurations include, but are not limited to: water-soluble polymers, e.g., water-soluble cellulosic materials, surfactants, salts, etc. Another example of a multilayer protective barrier is shown in the ingestible composition depicted in FIG. 5B. In FIG. 5B, ingestible composition 51 includes the IEM device 10 sandwiched between first and second multilayer protective barriers 53 and 55. Each multilayer protective barrier 53 and 55 is made up of a first layer 57 of a protective material, e.g., as described above, and a second layer 59 of a disrupting material that is more soluble that the protective material, e.g., as described above.

While the above examples were described in terms of the second material being more soluble than the protective material in an aqueous medium, as summarized above, other pairing of materials may also be employed. For example, the second disrupting material may have physical properties that differ from the protective material upon contact with the aqueous medium. Different physical properties may include water absorption, gas evolution, etc. For example, the second material may be a disrupting hydrogel which swells upon contact with an aqueous medium. Hydrogel materials of interest include, but are not limited to: pharmaceutically acceptable polymeric hydrogels, such as but not limited to: maltodextrin polymers comprising the formula (C₆H₁₂O₅)ₘ•H₂O, wherein m is 3 to 7,500, and the maltodextrin polymer comprises a 500 to 1,250,000 number-average molecular weight; a poly(alkylene oxide) represented by poly(ethylene oxide) and poly(propylene oxide) having a 50,000 to 750,000 weight-average molecular weight, e.g., by a poly(ethylene oxide) of at least one of 100,000, 200,000, 300,000, or 400,000 weight-average molecular weights; an alkali carboxyalkylcellulose, wherein the alkali is sodium, lithium, potassium or calcium, and alkyl is 1 to 5 carbons such as methyl, ethyl, propyl or butyl of 10,000 to 175,000 weight-average molecular weight; and a copolymer of ethylene-acrylic acid, including methacrylic and ethacrylic acid of 10,000 to 1,500,000 number-average molecular weight. Alternatively, the second disrupting material may be a material that is physiologically acceptable and produces a gas upon contact with an aqueous medium. Examples of such disrupting materials include materials that produce CO₂ upon contact with an aqueous medium, such as bicarbonate salts, e.g., sodium bicarbonate and potassium bicarbonate. In yet other embodiments, the second disrupting material may be a material that solubilizes the protective material, e.g., an enzyme that hydrolyzes the lipid protective material, such as described above.

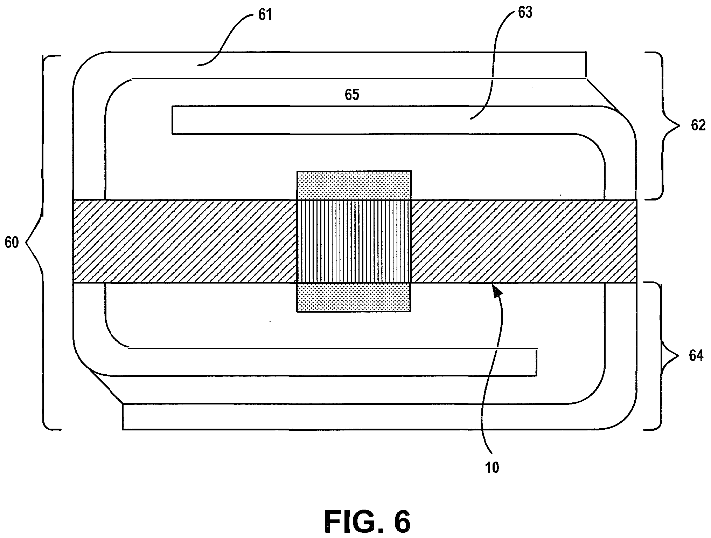

Multilayer configurations of interest also include overlapping, e.g., inter-digitated, configurations, such as depicted in FIG. 6. In FIG. 6, an ingestible composition 60 includes the IEM device 10 sandwiched between first and second protective barriers 62 and 64. Each protective barrier 62 and 64 includes first and second overlapping barrier layers 61 and 63 of a protective material separated from each other by second disrupting material 65. In the configuration shown in FIG. 6, each barrier layer 61 and 63 is secured at one end to the edge of the skirt component of the IEM 10.

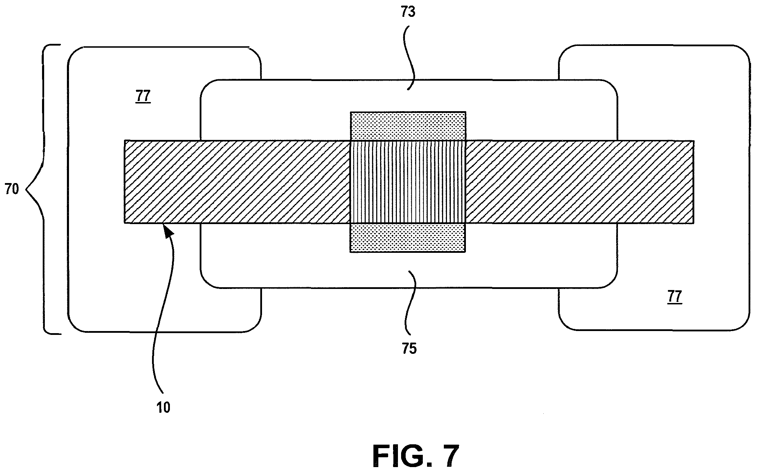

Another overlapping multilayer configuration is shown in FIG. 7. In FIG. 7, an ingestible composition 70 includes the IEM device 10 present between two opposing layers 73 and 75 of a first material. In addition, the edges of these opposing layers are capped with a second material 77. In composition 70, capping second material 77 has an annular configuration (e.g., having an outer diameter ranging from 5 mm to 8 mm and an inner diameter ranging from 2 mm to 5 mm) which partially overlaps the layers 73 and 75, and also caps the edge of the IEM skirt. In these configurations, the first and second materials may have different melting temperatures, e.g., the first material may have a melting temperature that is less than the melting temperature of the second material, and in some instances melts below 45°C. The differential in melting temperatures may vary, and in some instances ranges from 1 to 25 °C, such as 2 to 20 °C, including 5 to 15 °C. Any convenient pairs of materials may be employed for the first and second materials, where the pairs of materials may be the same or different types of materials, e.g., a protective material and solubilizing material, two types of lipids having different melting points, etc. Specific material pairings of interest include, but are not limited to: low-melting point materials, such as low-melting point lipids (e.g., lipids that melt below 45°C) and modified lipids/waxes; waxes and soluble polymers, and the like.

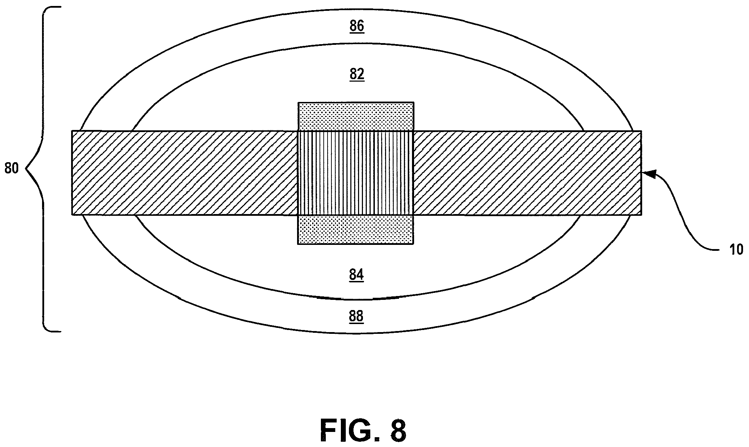

Yet another overlapping multilayer configuration is shown in FIG. 8. In FIG. 8, an ingestible composition 80 includes the IEM device 10 present between two opposing layers 82 and 84 of a first material. In addition, each of these opposing layers is further fully covered by second layers 86 and 88 made up of a second material. In these configurations, the first and second materials may have different melting temperatures, e.g., the first material may have a melting temperature that is less than the melting temperature of the second material. The differential in melting temperatures may vary, and in some instances ranges from 1 to 25 °C, such as 2 to 20 °C, including 5 to 15 °C. Any convenient pairs of materials may be employed for the first and second materials, where the pairs of materials may be the same or different types of materials, e.g., a protective material and solubilizing material, two types of lipids having different melting points, etc.

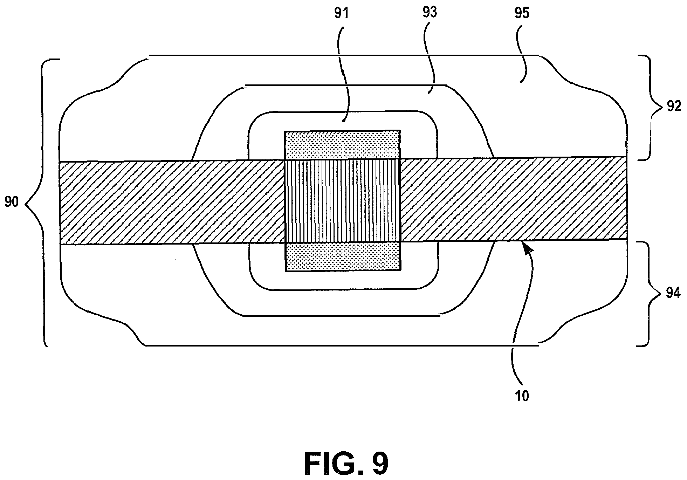

Yet another multilayer configuration showing protective barriers is depicted in FIG. 9. In FIG. 9, the ingestible composition is an example of compositions where the barrier is a multilayer structure of 2 or more distinct layers. In the particular embodiment depicted in FIG. 9, the protective barrier is made up of three layers. In FIG. 9, an ingestible composition 90 includes the IEM device 10 sandwiched between two protective barriers 92 and 94. Each protective barrier 92 and 94 includes three distinct layers (e.g., an IEM proximal layer, an intervening layer 93, and an outer layer 95). The IEM proximal layer 91 is made up of a protective material, e.g., as described above. The intervening layer 93 comprises a protective layer solubilizing material, e.g., an enzyme, surfactant, etc. The outer layer 95 comprises a water soluble layer, such as HPMC, HPC, e.g., described above.

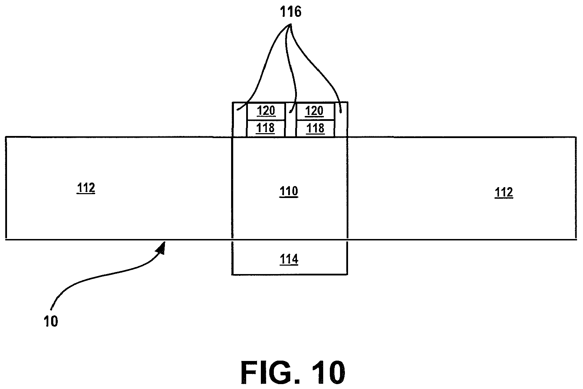

The protective barrier may also be a galvanic protective barrier. By "galvanic" is meant that the barrier material is one that is disrupted by galvanic corrosion upon immersion of the ingestible composition in a conducting fluid, e.g., stomach fluid. Galvanic protective barriers of interest include at least a protective metal. Protective metals of interest include those metals which are edible and have a water-sensitivity that is less than the sensitivity of the dissimilar material which they are intended to protect, e.g., CuCl. Specific protective metals of interest include magnesium, iron, copper, silver, etc. Where desired, a galvanic reaction initiator metal may be in contact with at least a portion of the protective metal, e.g., present along one or more edges (including the entire periphery of the protective metal), present in a region of the protective metal, etc. The galvanic reaction initiator metal is one that causes galvanic corrosion of the protective metal upon immersion in a conducting fluid, wherein galvanic reaction initiator metals of interest are ones that have a higher reduction potential than the protective metal. Examples of galvanic reaction initiator metals of interest include gold, platinum, etc. Any convenient configuration of the protective metal and the galvanic reaction initiator metal may be employed. FIG. 10 provides a view of an ingestible component that includes a galvanic protection layer according to one embodiment of the disclosure. In FIG. 10, the IEM device 10 includes integrated circuit 110 and membrane 112. Also shown is second dissimilar material 114, e.g., magnesium, on the bottom side of integrated circuit 100. On the top side of integrated circuit 110 are two regions of a first dissimilar material 118, e.g., CuCI. Separating the regions of the first dissimilar material 118 are walls of a galvanic reaction initiator metal 116, e.g., gold. Covering the layers of first dissimilar material 118 are metal protection layers 120, which are defect free layers that seal the first dissimilar material from the environment. The structure shown in FIG. 10 may be fabricated using any convenient protocol, e.g., by first forming wells on the top surface of integrated circuit 110 as defined by walls of the galvanic reaction initiator material 116, then depositing the first dissimilar material 118 in the two wells and finally depositing the layer of protective metal 120 over the layers of deposited first dissimilar material.

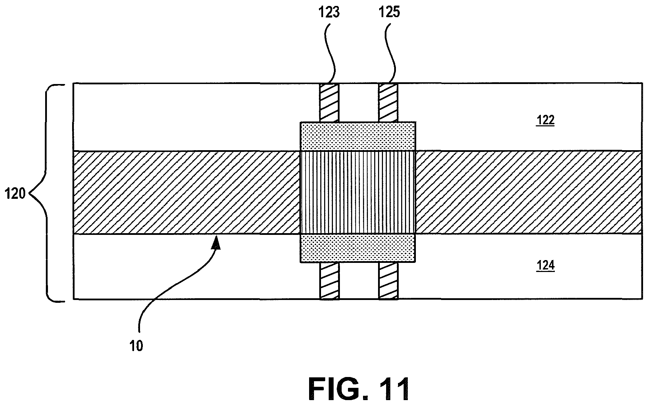

In some instances, the protective barrier is configured to provide aqueous liquid passage through the protective barrier upon contact of ingestible composition with an aqueous liquid. For example, the protective barrier may include one or more liquid passageways, which passageways may be filled (e.g., sealed (e.g., plugged) with a material that readily dissolves upon contact with an aqueous liquid medium. An example of such an ingestible composition is depicted in FIG. 11. In FIG. 11, an ingestible composition 120 includes the IEM device 10 sandwiched between first and second protective barriers, 122 and 124. The protective barriers 122 and 124 each include liquid passageways 123 and 125. The diameter of the passageways may vary, ranging in some instances from 0.01 to 0.5 mm, such as 0.01 to 0.05 mm. The length of the passageways may also vary, ranging in some instances from 1 to 10 mm, such as 2 to 5 mm. The passageways may have a linear or non-linear configuration, as desired. The passageways may be filled with a material serves to seal the IEM device 10 from a gaseous environment of the ingestible composition but that readily dissolves upon contact with an aqueous medium, thereby providing liquid access to the IEM device 10. Examples of such materials include any of the soluble materials listed above, e.g., salts, surfactants, etc. Where desired, such liquid passageways may be included in any of the protective barriers described above, e.g., as depicted in FIGS. 1 to 9.

In some instances, the protective barrier is configured to be disruptable by a device, e.g., the IEM device 10, present in the composition. For example, the protective barrier may include a material which melts in response to initial temperature changes produced upon the initial activation of the IEM device 10, such that the initial IEM activation enhances the disruption of the protective barrier. Examples of such materials include, but are not limited to, low melting point lipids, e.g., and the like. Alternative, the protective barrier or a component thereof may be a material that is responsive (e.g., in terms of changing dimension) to a voltage change caused by the IEM device 10, where examples of such materials include conductive polymers, such as ionomers, e.g., sulfonated tetrafluoroethylene based fluoropolymer-copolymer.

Instead of or in addition to the protective barrier, e.g., as described above, the ingestible composition may include other types of water-vapor desensitizers. Other types of water-vapor desensitizers include water vapor sequestering materials, e.g., desiccants. A variety of different types of desiccant materials may be employed, where representative desiccant materials include solid materials, e.g., beads and strips or blocks of desiccant material, etc. Representative materials that may be employed as desiccants include, but are not limited to: molecular sieve, silica gel, CaSO₄, CaO, magnesium aluminum-metasilicate, and the like. Incorporated into the desiccant material may be an indicator that provides a detectable single, e.g., color change, that can be used to determine the remaining capacity of the desiccant, e.g., to determine whether or not a desiccant has reached capacity with respect to the amount of water that it can sequester. Indicator compounds of interest include, but are not limited to: CoCl₂ and the like.

Also of interest are barrier compositions that include an amount of a water/O₂ scavenger material. Examples of such materials include, but are not limited to: mercapto compounds, e.g., mercaptoalkanols, such as 3-mercapto-3-methyl-butan-1-ol, 3-mercapto-2-methyl-propan-1-ol and 2-Mercaptopyridine; BHA, BHT, benzothiazole, etc. When present, the amount of such compounds may vary, ranging in some instances from 1 ppb to 1%, such as 0.01% to 0.5%.

### SYSTEMS

Also provided are systems that include an ingestible composition or device, e.g., an IEM, and a detection component, e.g., in the form of a receiver. As discussed above, the ingestible composition comprises a shelf-life stability component and an ingestible component associated with the shelf-life stability component. Receivers of interest are those configured to detect, e.g., receive, a communication from the ingestible composition or device, e.g., RFID ingestible device, IEM, etc. The signal detection component may vary significantly depending on the nature of the communication that is generated by the ingestible device. As such, the receiver may be configured to receive a variety of different types of signals, including but not limited to: RF signals, magnetic signals, conductive (near field) signals, acoustic signals, etc. In certain aspects, the receiver is configured to receive a signal conductively from an IEM, such that the two components use the body of the patient as a communication medium. As such, communication that is transferred between IEM and the receiver travels through the body, and requires the body as the conduction medium. The IEM communication may be transmitted through and received from the skin and other body tissues of the subject body in the form of electrical alternating current (a.c.) voltage signals that are conducted through the body tissues. This communication protocol has the advantage that the receivers may be adaptably arranged at any desired location on the body of the subject, whereby the receivers are automatically connected to the required electrical conductor for achieving the signal transmission, e.g., the signal transmission is carried out through the electrical conductor provided by the skin and other body tissues of the subject.

The receivers of interest include external, semi-implantable, and implantable receivers. In external aspects, the receiver is *ex vivo*, by which is meant that the receiver is present outside of the body during use. Examples include wearable patches, e.g., adhesive patches, torso bands, wrist(s) or arm bands, jewelry, apparel, mobile devices such as phones, attachments to mobile devices, etc. Where the receiver is implanted, the receiver is *in vivo.* Examples include cardiac can and leads, under-the-skin implants, etc. Semi-implantable devices include those designed to be partially implanted under the skin.

In certain aspects, the receiver may be configured to provide data associated with a received signal to a location external to the subject. For example, the receiver may be configured to provide data to an external data receiver, e.g., which may be in the form of a monitor (such as a bedside monitor), a computer, a personal digital assistant (PDA), phone, messaging device, smart phone, etc. The receiver may be configured to retransmit data of a received communication to the location external to the subject. Alternatively, the receiver may be configured to be interrogated by an external interrogation device to provide data of a received signal to an external location.

Receivers may be configured variously, e.g., with various signal receiving elements, such as electrodes, various integrated circuit components, one or more power components (such as power receivers or batteries), signal transmission components, housing components, etc.

In one aspect, for example, the receiver includes one or more of: a high power-low power module; an intermediary module; a power supply module configured to activate and deactivate one or more power supplies to a high power processing block; a serial peripheral interface bus connecting master and slave blocks; and a multi-purpose connector, as further described in PCT application serial No. PCT/US2009/068128 published as WO2010/075115, *infra.*

Receivers of interest include, but are not limited to, those receivers disclosed in: PCT application serial no. PCT/US2006/016370 published as WO 2006/116718; PCT application serial no. PCT/US2008/52845 published as WO 2008/095183; PCT application serial no. PCT/US2007/024225 published as WO 2008/063626; PCT application serial no. PCT/US2008/085048 published as WO 009/070773; PCT application serial no. PCT/US2009/068128 published as WO2010/075115; and US provisional application serial no. 61/510,434 filed on July 21, 2011 the disclosures of which applications (and particularly receiver components thereof) are herein incorporated by reference.

Systems of the disclosure may include an external device which is distinct from the receiver (which may be implanted or topically applied in certain aspects), where this external device provides a number of functionalities. Such an apparatus can include the capacity to provide feedback and appropriate clinical regulation to the patient. Such a device can take any of a number of forms. By example, the device can be configured to sit on the bed next to the patient, e.g., a bedside monitor. Other formats include, but are not limited to, PDAs, phones, such as smart phones, computers, etc. The device can read out the information described in more detail in other sections of the subject patent application, both from pharmaceutical ingestion reporting and from physiological sensing devices, such as is produced internally by a pacemaker device or a dedicated implant for detection of the pill. The purpose of the external apparatus is to get the data out of the patient and into an external device. One feature of the external apparatus is its ability to provide pharmacologic and physiologic information in a form that can be transmitted through a transmission medium, such as a telephone line, to a remote location such as a clinician or to a central monitoring agency.

### MANUFACTURING METHODS

Also provided are methods of manufacturing ingestible compositions, e.g, as described herein. Aspects of the methods include combining a minimally dimensioned component such as an ingestible component (which may or may not include a device, such as an IEM) and a shelf-life stability component, e.g., as described above, in a manner sufficient to produce a shelf-life stable ingestible composition. Any convenient manufacturing protocol may be employed, where protocols of interest include both manual and automated protocols, as well as protocols that include both manual and automated steps. Protocols of interest that find use in various aspects of the fabrication methods described herein include lamination, molding, pressing, extrusion, stamping, coating (such as spray coating and dipping), etc. In some instances, fabrication protocols of interest include, but not limited to, those protocols disclosed in: PCT application serial no. PCT/US2010/020142 published as WO 2010/080765; PCT application serial no. PCT/US2006/016370 published as WO 2006/116718; and PCT application serial no. PCT/US08/77753 published as WO 2009/042812 are herein incorporated by reference.

Aspects of the fabrication protocols include stably associating the ingestible component with the shelf-life stability component. By "stably associating" is meant that the ingestible component and shelf-life stability component, e.g., protective barrier, do not separate from each other, at least until administered to the subject in need thereof, e.g., by ingestion. Any convenient approach for stably associating the ingestible component and the shelf-life stability component may be employed.

Where the ingestible component is positioned between two protective barrier components, e.g., as illustrated in FIGS. 2 to 5B, a protocol in which pre-fabricated protective barrier components may be employed. In such a protocol, the ingestible component may be positioned between the two pre-fabricated protective barrier components, e.g., in a manner sufficient to seal the ingestible component between the pre-fabricated protective barrier components. Where desired, an adhesive may be employed to secure the two protective barrier components together.

In a variation of the above protocol, a fabrication process may be one in which the protective barrier components are fabricated at the same time that the ingestible component is stably associated therewith. For example, a molding process may be employed where a protective barrier component precursor material, e.g., a liquid lipid/carrier material blend (such as described above), is positioned in a mold, followed by placement of an ingestible component (e.g., an IEM) on the precursor material and then placement of an additional amount of precursor material on top of the ingestible component. Temperature modulation may be employed where appropriate, e.g., where the precursor material is a liquid at body temperature but a solid at room temperature. Following solidification of the precursor material, the resultant final product may be removed from the mold.

In yet another fabrication protocol of interest, a stamping protocol may be employed. For example, an ingestible component may be positioned between two sheets of a prefabricated multilayer protective barrier component, such as a sheet of a protective barrier component that includes a soluble layer and an insoluble layer, e.g., as described above. Once positioned between the two sheets, a stamping tool may be used to stamp and seal the two sheets around the ingestible component in a manner that encases the ingestible component in a sealed multilayer protective barrier. The stamping tool may be configuration to produce a product having any convenient shape, such as a disc, etc. Where desired, temperature modulation may be employed in such protocols.

In yet another fabrication protocol of interest, a coating process may be employed to stably associate the ingestible component with the shelf-life stability component. For example, a premade ingestible component in the form of a tablet may be provided, e.g., as described in PCT application serial nos. PCT/US2010/020142 published as WO 2010/080765; PCT/US2006/016370 published as WO 2006/116718; and PCT/US08/77753 published as WO 2009/042812 (the disclosures of which are herein incorporated by reference). This premade ingestible component may then be spray coated with a liquid protective barrier precursor material (e.g., as described above). Following spray coating, the coating material may be allowed to harden (e.g., by maintaining the coated tablet at a suitable temperature, such as room temperature) to produce the desired product.

Where desired, aspects of the above described or other suitable protocols may be combined to produce a fabrication protocol. For example, a molding process may be employed to make a product and the product may be spray coated with a further material, such as a soluble material.

### METHODS OF USE

Aspects of the disclosure further include methods of using the compositions, such as those described above. Aspects of such methods include administering an ingestible composition to a subject, e.g., by self-administration or via the assistance of another, such as a health care practitioner. Such methods may include placing the ingestible composition in the mouth of a subject such that the subject swallows the ingestible composition. In this manner, the subject ingests the ingestible composition. Ingestible compositions may be employed with a variety of subjects. Generally such subjects are "mammals" or "mammalian," where these terms are used broadly to describe organisms which are within the class mammalia, including the orders carnivore (e.g., dogs and cats), rodentia (e.g., mice, guinea pigs, and rats), and primates (e.g., humans, chimpanzees, and monkeys). In certain aspects, the subjects may be humans.

Following ingestion, the methods may include receiving a signal emitted from an ingestible composition, such as an IEM comprising ingestible composition, e.g., at a receiver, such as described above. In some instances, the received signal is a conductively transmitted signal.

Ingestible composition may be employed in a variety of different applications. Applications of interest, in which the ingestible composition comprises an IEM include, but are not limited to: monitoring patient compliance with prescribed therapeutic regimens; tailoring therapeutic regimens based on the patient compliance; monitoring the patient compliance in clinical trials; monitoring usage of controlled substances; monitoring the occurrence of a personal event of interest, such as the onset of symptoms, etc., and the like. Applications of interest are further described in PCT application serial no. PCT/US2006/016370 published as WO/2006/116718; PCT application serial no. PCT/US2007/082563 published as WO/2008/052136; PCT application serial no. PCT/US2007/024225 published as WO/2008/063626; PCT application serial no. PCT/US2007/022257 published as WO/2008/066617; PCT application serial no. PCT/US2008/052845 published as WO/2008/095183; PCT application serial no. PCT/US2008/053999 published as WO/2008/101107; PCT application serial no. PCT/US2008/056296 published as WO/2008/112577; PCT application serial no. PCT/US2008/056299 published as WO/2008/112578; and PCT application serial no. PCT/US2008/077753 published as WO 2009/042812; the disclosures of which applications is herein incorporated by reference.

### KITS

Also provided are kits that include one or more ingestible compositions, such as described above. In those aspects having a plurality of ingestible compositions, the ingestible compositions may be packaged in a single container, e.g., a single tube, bottle, vial, and the like, or one or more dosage amounts may be individually packaged such that certain kits may have more than one container of ingestible compositions. In certain aspects the kits may also include a receiver, such as reviewed above. In certain aspects, the kits may also include an external monitor device, e.g., as described above, which may provide for communication with a remote location, e.g., a doctor's office, a central facility etc., which obtains and processes data obtained about the usage of the composition.

The subject kits may also include instructions for how to practice the subject methods using the components of the kit. The instructions may be recorded on a suitable recording medium or substrate. For example, the instructions may be printed on a substrate, such as paper or plastic, etc. As such, the instructions may be present in the kits as a package insert, in the labeling of the container of the kit or components thereof (e.g., associated with the packaging or sub-packaging) etc. In other aspects, the instructions are present as an electronic storage data file present on a suitable computer readable storage medium, e.g. CD-ROM, diskette, etc. In yet other aspects, the actual instructions are not present in the kit, but means for obtaining the instructions from a remote source, e.g. via the internet, are provided. An example of this aspect is a kit that includes a web address where the instructions can be viewed and/or from which the instructions can be downloaded. As with the instructions, this means for obtaining the instructions is recorded on a suitable substrate.

Some or all components of the subject kits may be packaged in suitable packaging to maintain sterility. In many aspects of the subject kits, the components of the kit are packaged in a kit containment element to make a single, easily handled unit, where the kit containment element, e.g., box or analogous structure, may or may not be an airtight container, e.g., to further preserve the sterility of some or all of the components of the kit.

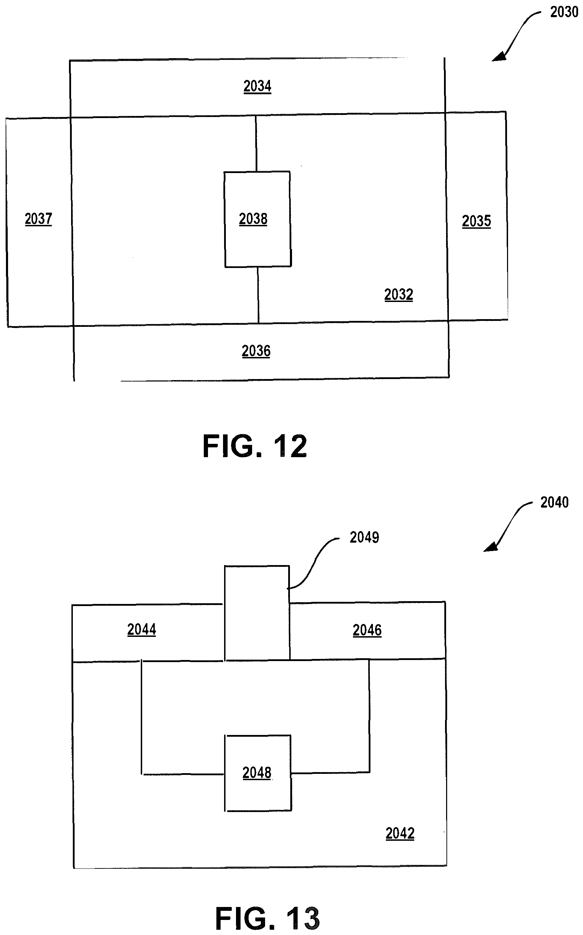

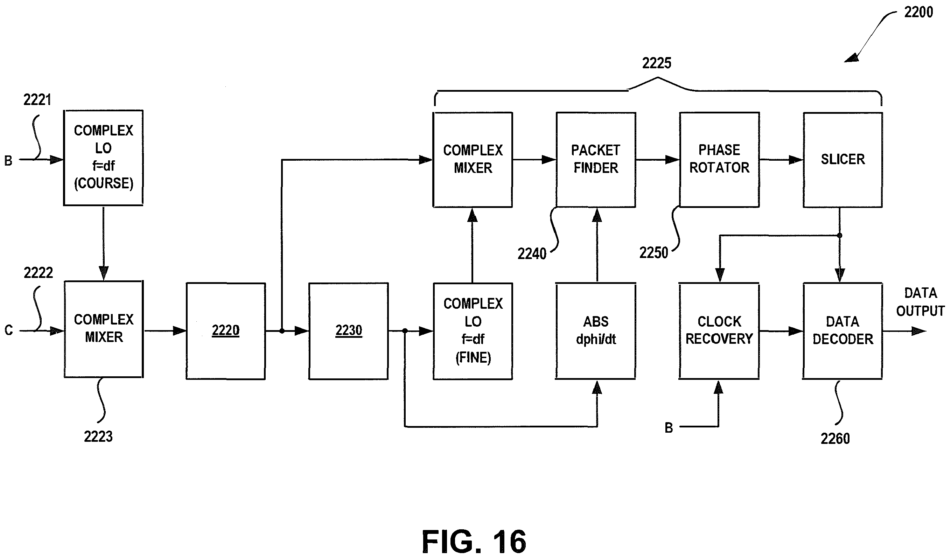

Various enabling aspects of the IEM are illustrated in FIGs. 12-15 below. It is appreciated that the IEM may be a system which comprises a partial power source that can be activated when in contact with conductive liquid and is capable of controlling conductance to mark an event. In the instance where the system is used with the product that is ingested by the living organism, when the product that includes the system is taken or ingested, the device comes into contact with the conducting liquid of the body. When the system of the present disclosure comes into contact with the body fluid, a voltage potential is created and the system is activated. A portion of the power source is provided by the device, while another portion of the power source is provided by the conducting fluid. That is, once ingested, the system comes into contact with body liquids and the system is activated. The system uses the voltage potential difference to power up and thereafter modulates conductance to create a unique and identifiable current signature. Upon activation, the system controls the conductance and, hence, current flow to produce the current signature. In addition, various enabling aspects of the receiver/detector are illustrated in FIGs. 16-21 below.

With reference to FIG. 12, there is shown one aspect of an ingestible device event indicator system with dissimilar metals positioned on opposite ends as system 2030. The system 2030 can be used in association with any pharmaceutical product, as mentioned above, to determine when a patient takes the pharmaceutical product. As indicated above, the scope of the present disclosure is not limited by the environment and the product that is used with the system 2030. For example, the system 2030 may be placed within a capsule and the capsule is placed within the conducting liquid. The capsule would then dissolve over a period of time and release the system 2030 into the conducting liquid. Thus, in one aspect, the capsule would contain the system 2030 and no product. Such a capsule may then be used in any environment where a conducting liquid is present and with any product. For example, the capsule may be dropped into a container filled with jet fuel, salt water, tomato sauce, motor oil, or any similar product. Additionally, the capsule containing the system 2030 may be ingested at the same time that any pharmaceutical product is ingested in order to record the occurrence of the event, such as when the product was taken.

In the specific example of the system 2030 combined with the pharmaceutical product, as the product or pill is ingested, the system 2030 is activated. The system 2030 controls conductance to produce a unique current signature that is detected, thereby signifying that the pharmaceutical product has been taken. The system 2030 includes a framework 2032. The framework 2032 is a chassis for the system 2030 and multiple components are attached to, deposited upon, or secured to the framework 2032. In this aspect of the system 2030, a digestible material 2034 is physically associated with the framework 2032. The material 2034 may be chemically deposited on, evaporated onto, secured to, or built-up on the framework all of which may be referred to herein as "deposit" with respect to the framework 2032. The material 2034 is deposited on one side of the framework 2032. The materials of interest that can be used as material 2034 include, but are not limited to: Cu or Cul. The material 2034 is deposited by physical vapor deposition, electrodeposition, or plasma deposition, among other protocols. The material 2034 may be from about 0.05 to about 500 .mu.m thick, such as from about 5 to about 100 .mu.m thick. The shape is controlled by shadow mask deposition, or photolithography and etching. Additionally, even though only one region is shown for depositing the material, each system 2030 may contain two or more electrically unique regions where the material 2034 may be deposited, as desired.

At a different side, which is the opposite side as shown in FIG. 12, another digestible material 2036 is deposited, such that materials 2034 and 2036 are dissimilar. Although not shown, the different side selected may be the side next to the side selected for the material 2034. The scope of the present disclosure is not limited by the side selected and the term "different side" can mean any of the multiple sides that are different from the first selected side. Furthermore, even though the shape of the system is shown as a square, the shape maybe any geometrically suitable shape. Material 2034 and 2036 are selected such that they produce a voltage potential difference when the system 2030 is in contact with conducting liquid, such as body fluids. The materials of interest for material 2036 include, but are not limited to: Mg, Zn, or other electronegative metals. As indicated above with respect to the material 2034, the material 2036 may be chemically deposited on, evaporated onto, secured to, or built-up on the framework. Also, an adhesion layer may be necessary to help the material 2036 (as well as material 2034 when needed) to adhere to the framework 2032. Typical adhesion layers for the material 2036 are Ti, TiW, Cr or similar material. Anode material and the adhesion layer may be deposited by physical vapor deposition, electrodeposition or plasma deposition. The material 2036 may be from about 0.05 to about 500 .mu.m thick, such as from about 5 to about 100 .mu.m thick. However, the scope of the present disclosure is not limited by the thickness of any of the materials nor by the type of process used to deposit or secure the materials to the framework 2032.

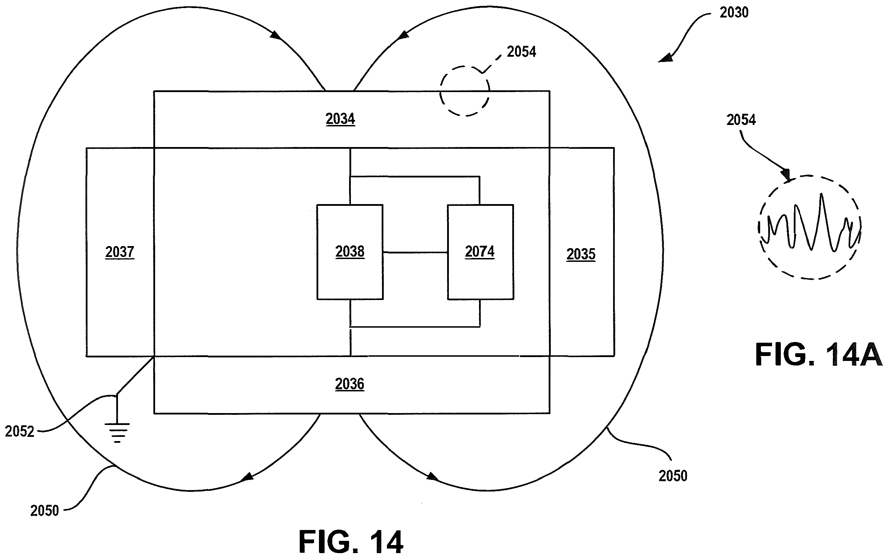

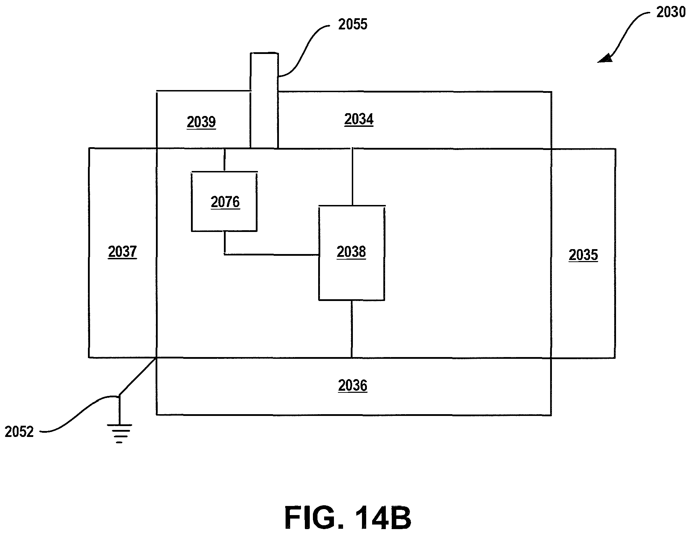

Thus, when the system 2030 is in contact with the conducting liquid, a current path, an example is shown in FIG. 14, is formed through the conducting liquid between materials 2034 and 2036. A control device 2038 is secured to the framework 2032 and electrically coupled to the materials 2034 and 2036. The control device 2038 includes electronic circuitry, for example control logic that is capable of controlling and altering the conductance between the materials 2034 and 2036.

The voltage potential created between the materials 2034 and 2036 provides the power for operating the system as well as produces the current flow through the conducting fluid and the system. In one aspect, the system operates in direct current mode. In an alternative aspect, the system controls the direction of the current so that the direction of current is reversed in a cyclic manner, similar to alternating current. As the system reaches the conducting fluid or the electrolyte, where the fluid or electrolyte component is provided by a physiological fluid, e.g., stomach acid, the path for current flow between the materials 2034 and 2036 is completed external to the system 2030; the current path through the system 2030 is controlled by the control device 2038. Completion of the current path allows for the current to flow and in turn a receiver can detect the presence of the current and recognize that the system 2030 has been activated and the desired event is occurring or has occurred.

In one aspect, the two materials 2034 and 2036 are similar in function to the two electrodes needed for a direct current power source, such as a battery. The conducting liquid acts as the electrolyte needed to complete the power source. The completed power source described is defined by the physical chemical reaction between the materials 2034 and 2036 of the system 2030 and the surrounding fluids of the body. The completed power source may be viewed as a power source that exploits reverse electrolysis in an ionic or a conductive solution such as gastric fluid, blood, or other bodily fluids and some tissues. Additionally, the environment may be something other than a body and the liquid may be any conducting liquid. For example, the conducting fluid may be salt water or a metallic based paint.

In certain aspects, these two materials are shielded from the surrounding environment by an additional layer of material. Accordingly, when the shield is dissolved and the two dissimilar materials are exposed to the target site, a voltage potential is generated.

Referring again to FIG. 12, the materials 2034 and 2036 provide the voltage potential to activate the control device 2038. Once the control device 2038 is activated or powered up, the control device 2038 can alter conductance between the materials 2034 and 2036 in a unique manner. By altering the conductance between materials 2034 and 2036, the control device 2038 is capable of controlling the magnitude of the current through the conducting liquid that surrounds the system 2030. This produces a unique current signature that can be detected and measured by a receiver, which can be positioned internal or external to the body. In addition to controlling the magnitude of the current path between the materials, non-conducting materials, membrane, or "skirt" are used to increase the "length" of the current path and, hence, act to boost the conductance path, as disclosed in the U.S. patent application Ser. No. 12/238,345 filed Sep. 25, 2008, published 2009-0082645, and entitled, "In-Body Device with Virtual Dipole Signal Amplification", the entire content of which is incorporated herein by reference. Alternatively, throughout the disclosure herein, the terms "non-conducting material", "membrane", and "skirt" are interchangeably with the term "current path extender" without impacting the scope or the present aspects and the claims herein. The skirt, shown in portion at 2035 and 2037, respectively, may be associated with, e.g., secured to, the framework 2032. Various shapes and configurations for the skirt are contemplated as within the scope of the present disclosure. For example, the system 2030 may be surrounded entirely or partially by the skirt and the skirt maybe positioned along a central axis of the system 2030 or off-center relative to a central axis. Thus, the scope of the present disclosure as claimed herein is not limited by the shape or size of the skirt. Furthermore, in other aspects, the materials 2034 and 2036 may be separated by one skirt that is positioned in any defined region between the materials 2034 and 2036.

Referring now to FIG. 13, in another aspect of an ingestible device is shown in more detail as system 2040. The system 2040 includes a framework 2042. The framework 2042 is similar to the framework 2032 of FIG. 12. In this aspect of the system 2040, a digestible or dissolvable material 2044 is deposited on a portion of one side of the framework 2042. At a different portion of the same side of the framework 2042, another digestible material 2046 is deposited, such that materials 2044 and 2046 are dissimilar. More specifically, material 2044 and 2046 are selected such that they form a voltage potential difference when in contact with a conducting liquid, such as body fluids. Thus, when the system 2040 is in contact with and/or partially in contact with the conducting liquid, then a current path, an example is shown in FIG. 14, is formed through the conducting liquid between materials 2044 and 2046. A control device 2048 is secured to the framework 2042 and electrically coupled to the materials 2044 and 2046. The control device 2048 includes electronic circuitry that is capable of controlling part of the conductance path between the materials 2044 and 2046. The materials 2044 and 2046 are separated by a non-conducting skirt 2049. Various examples of the skirt 2049 are disclosed in U.S. Provisional Application No. 61/173,511 filed on Apr. 28, 2009 and entitled "HIGHLY RELIABLE INGESTIBLE EVENT MARKERS AND METHODS OF USING SAME" and U.S. Provisional Application No. 61/173,564 filed on Apr. 28, 2009 and entitled "INGESTIBLE EVENT MARKERS HAVING SIGNAL AMPLIFIERS THAT COMPRISE AN ACTIVE AGENT"; as well as U.S. application Ser. No. 12/238,345 filed Sep. 25, 2008, published 2009-0082645, entitled "IN-BODY DEVICE WITH VIRTUAL DIPOLE SIGNAL AMPLIFICATION"; the entire disclosure of each is incorporated herein by reference.

Once the control device 2048 is activated or powered up, the control device 2048 can alter conductance between the materials 2044 and 2046. Thus, the control device 2048 is capable of controlling the magnitude of the current through the conducting liquid that surrounds the system 2040. As indicated above with respect to system 2030, a unique current signature that is associated with the system 2040 can be detected by a receiver to mark the activation of the system 2040. In order to increase the "length" of the current path the size of the skirt 2049 is altered. The longer the current path, the easier it may be for the receiver to detect the current.

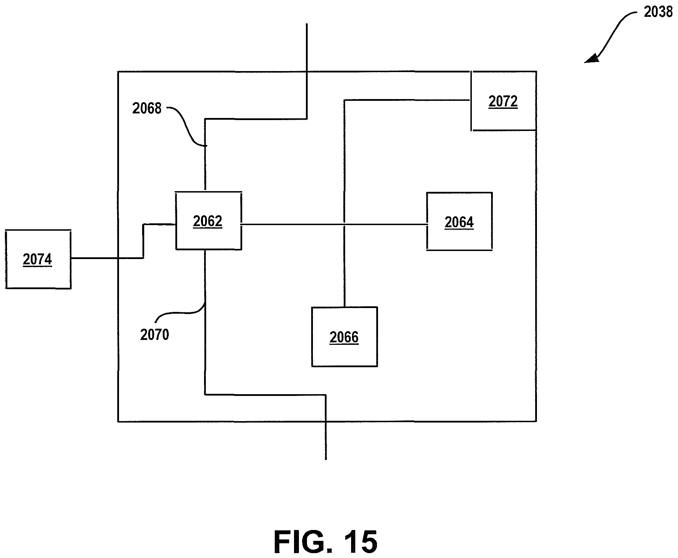

Referring now to FIG. 14, the system 2030 of FIG. 12 is shown in an activated state and in contact with conducting liquid. The system 2030 is grounded through ground contact 2052. The system 2030 also includes a sensor module 2074, which is described in greater detail with respect to FIG. 15. Ion or current paths 2050 form between material 2034 to material 2036 through the conducting fluid in contact with the system 2030. The voltage potential created between the material 2034 and 2036 is created through chemical reactions between materials 2034/2036 and the conducting fluid.

FIG. 14A shows an exploded view of the surface of the material 2034. The surface of the material 2034 is not planar, but rather an irregular surface 2054 as shown. The irregular surface 2054 increases the surface area of the material and, hence, the area that comes in contact with the conducting fluid.

In one aspect, at the surface of the material 2034, there is chemical reaction between the material 2034 and the surrounding conducting fluid such that mass is released into the conducting fluid. The term "mass" as used herein refers to protons and neutrons that form a substance. One example includes the instant where the material is CuCl and when in contact with the conducting fluid, CuCl becomes Cu (solid) and Cl.sup.- in solution. The flow of ions into the conduction fluid is depicted by the ion paths 2050. In a similar manner, there is a chemical reaction between the material 2036 and the surrounding conducting fluid and ions are captured by the material 2036. The release of ions at the material 2034 and capture of ion by the material 2036 is collectively referred to as the ionic exchange. The rate of ionic exchange and, hence the ionic emission rate or flow, is controlled by the control device 2038. The control device 2038 can increase or decrease the rate of ion flow by altering the conductance, which alters the impedance, between the materials 2034 and 2036. Through controlling the ion exchange, the system 2030 can encode information in the ionic exchange process. Thus, the system 2030 uses ionic emission to encode information in the ionic exchange.

The control device 2038 can vary the duration of a fixed ionic exchange rate or current flow magnitude while keeping the rate or magnitude near constant, similar to when the frequency is modulated and the amplitude is constant. Also, the control device 2038 can vary the level of the ionic exchange rate or the magnitude of the current flow while keeping the duration near constant. Thus, using various combinations of changes in duration and altering the rate or magnitude, the control device 2038 encodes information in the current flow or the ionic exchange. For example, the control device 2038 may use, but is not limited to any of the following techniques namely, Binary Phase-Shift Keying (PSK), Frequency modulation, Amplitude modulation, on-off keying, and PSK with on-off keying.

As indicated above, the various aspects disclosed herein, such as systems 2030 and 2040 of FIGS. 12 and 13, respectively, include electronic components as part of the control device 2038 or the control device 2048. Components that may be present include but are not limited to: logic and/or memory elements, an integrated circuit, an inductor, a resistor, and sensors for measuring various parameters. Each component may be secured to the framework and/or to another component. The components on the surface of the support may be laid out in any convenient configuration. Where two or more components are present on the surface of the solid support, interconnects may be provided.

As indicated above, the system, such as system 2030 and 2040, control the conductance between the dissimilar materials and, hence, the rate of ionic exchange or the current flow. Through altering the conductance in a specific manner the system is capable of encoding information in the ionic exchange and the current signature. The ionic exchange or the current signature is used to uniquely identify the specific system. Additionally, the systems 2030 and 2040 are capable of producing various different unique exchanges or signatures and, thus, provide additional information. For example, a second current signature based on a second conductance alteration pattern may be used to provide additional information, which information may be related to the physical environment. To further illustrate, a first current signature may be a very low current state that maintains an oscillator on the chip and a second current signature may be a current state at least a factor of ten higher than the current state associated with the first current signature.

Referring now to FIG. 15, a block diagram representation of the control device 2038 is shown. The device 2038 includes a control module 2062, a counter or clock 2064, and a memory 2066. Additionally, the device 2038 is shown to include a sensor module 2072 as well as the sensor module 2074, which was referenced in FIG. 14. The control module 2062 has an input 2068 electrically coupled to the material 2034 and an output 2070 electrically coupled to the material 2036. The control module 2062, the clock 2064, the memory 2066, and the sensor modules 2072/2074 also have power inputs (some not shown). The power for each of these components is supplied by the voltage potential produced by the chemical reaction between materials 2034 and 2036 and the conducting fluid, when the system 2030 is in contact with the conducting fluid. The control module 2062 controls the conductance through logic that alters the overall impedance of the system 2030. The control module 2062 is electrically coupled to the clock 2064. The clock 2064 provides a clock cycle to the control module 2062. Based upon the programmed characteristics of the control module 2062, when a set number of clock cycles have passed, the control module 2062 alters the conductance characteristics between materials 2034 and 2036. This cycle is repeated and thereby the control device 2038 produces a unique current signature characteristic. The control module 2062 is also electrically coupled to the memory 2066. Both the clock 2064 and the memory 2066 are powered by the voltage potential created between the materials 2034 and 2036.

The control module 2062 is also electrically coupled to and in communication with the sensor modules 2072 and 2074. In the aspect shown, the sensor module 2072 is part of the control device 2038 and the sensor module 2074 is a separate component. In alternative aspects, either one of the sensor modules 2072 and 2074 can be used without the other and the scope of the present disclosure is not limited by the structural or functional location of the sensor modules 2072 or 2074. Additionally, any component of the system 2030 may be functionally or structurally moved, combined, or repositioned without limiting the scope of the present disclosure as claimed. Thus, it is possible to have one single structure, for example a processor, which is designed to perform the functions of all of the following modules: the control module 2062, the clock 2064, the memory 2066, and the sensor module 2072 or 2074. On the other hand, it is also within the scope of the present disclosure to have each of these functional components located in independent structures that are linked electrically and able to communicate.

Referring again to FIG. 15, the sensor modules 2072 or 2074 can include any of the following sensors: temperature, pressure, pH level, and conductivity. In one aspect, the sensor modules 2072 or 2074 gather information from the environment and communicate the analog information to the control module 2062. The control module then converts the analog information to digital information and the digital information is encoded in the current flow or the rate of the transfer of mass that produces the ionic flow. In another aspect, the sensor modules 2072 or 2074 gather information from the environment and convert the analog information to digital information and then communicate the digital information to control module 2062. In the aspect shown in FIG. 14, the sensor modules 2074 is shown as being electrically coupled to the material 2034 and 2036 as well as the control device 2038. In another aspect, as shown in FIG. 15, the sensor module 2074 is electrically coupled to the control device 2038 at a connection. The connection acts as both a source for power supply to the sensor module 2074 and a communication channel between the sensor module 2074 and the control device 2038.

Referring now to FIG. 14B, the system 2030 includes a pH sensor module 2076 connected to a material 2039, which is selected in accordance with the specific type of sensing function being performed. The pH sensor module 2076 is also connected to the control device 2038. The material 2039 is electrically isolated from the material 2034 by a non-conductive barrier 2055. In one aspect, the material 2039 is platinum. In operation, the pH sensor module 2076 uses the voltage potential difference between the materials 2034/2036. The pH sensor module 2076 measures the voltage potential difference between the material 2034 and the material 2039 and records that value for later comparison. The pH sensor module 2076 also measures the voltage potential difference between the material 2039 and the material 2036 and records that value for later comparison. The pH sensor module 2076 calculates the pH level of the surrounding environment using the voltage potential values. The pH sensor module 2076 provides that information to the control device 2038. The control device 2038 varies the rate of the transfer of mass that produces the ionic transfer and the current flow to encode the information relevant to the pH level in the ionic transfer, which can be detected by a receiver. Thus, the system 2030 can determine and provide the information related to the pH level to a source external to the environment.

As indicated above, the control device 2038 can be programmed in advance to output a pre-defined current signature. In another aspect, the system can include a receiver system that can receive programming information when the system is activated. In another aspect, not shown, the switch 2064 and the memory 2066 can be combined into one device.

In addition to the above components, the system 2030 may also include one or other electronic components. Electrical components of interest include, but are not limited to: additional logic and/or memory elements, e.g., in the form of an integrated circuit; a power regulation device, e.g., battery, fuel cell or capacitor; a sensor, a stimulator, etc.; a signal transmission element, e.g., in the form of an antenna, electrode, coil, etc.; a passive element, e.g., an inductor, resistor, etc.

FIG. 16 provides a functional block diagram of how a receiver may implement a coherent demodulation protocol, according to one aspect of the disclosure. It should be noted that only a portion of the receiver is shown in FIG. 16. FIG. 16 illustrates the process of mixing the signal down to baseband once the carrier frequency (and carrier signal mixed down to carrier offset) is determined. A carrier signal 2221 is mixed with a second carrier signal 2222 at mixer 2223. A narrow low-pass filter 2220 is applied of appropriate bandwidth to reduce the effect of out-of-bound noise. Demodulation occurs at functional blocks 2225 in accordance with the coherent demodulation scheme of the present disclosure. The unwrapped phase 2230 of the complex signal is determined. An optional third mixer stage, in which the phase evolution is used to estimate the frequency differential between the calculated and real carrier frequency can be applied. The structure of the packet is then leveraged to determine the beginning of the coding region of the BPSK signal at block 2240. Mainly, the presence of the sync header, which appears as an FM porch in the amplitude signal of the complex demodulated signal is used to determine the starting bounds of the packet. Once the starting point of the packet is determined the signal is rotated at block 2250 on the IQ plane and standard bit identification and eventually decoded at block 2260.

In addition to demodulation, the transbody communication module may include a forward error correction module, which module provides additional gain to combat interference from other unwanted signals and noise. Forward error correction functional modules of interest include those described in PCT Application Serial No. PCT/US2007/024225 published as WO/2008/063626; the disclosure of which is herein incorporated by reference. In some instances, the forward error correction module may employ any convenient protocol, such as Reed-Solomon, Golay, Hamming, BCH, and Turbo protocols to identify and correct (within bounds) decoding errors.

Receivers of the disclosure may further employ a beacon functionality module. In various aspects, the beacon switching module may employ one or more of the following: a beacon wakeup module, a beacon signal module, a wave/frequency module, a multiple frequency module, and a modulated signal module.

The beacon switching module may be associated with beacon communications, e.g., a beacon communication channel, a beacon protocol, etc. For the purpose of the present disclosure, beacons are typically signals sent either as part of a message or to augment a message (sometimes referred to herein as "beacon signals"). The beacons may have well-defined characteristics, such as frequency. Beacons may be detected readily in noisy environments and may be used for a trigger to a sniff circuit, such as described below.

In one aspect, the beacon switching module may comprise the beacon wakeup module, having wakeup functionality. Wakeup functionality generally comprises the functionality to operate in high power modes only during specific times, e.g., short periods for specific purposes, to receive a signal, etc. An important consideration on a receiver portion of a system is that it be of low power. This feature may be advantageous in an implanted receiver, to provide for both small size and to preserve a long-functioning electrical supply from a battery. The beacon switching module enables these advantages by having the receiver operate in a high power mode for very limited periods of time. Short duty cycles of this kind can provide optimal system size and energy draw features.

In practice, the receiver may "wake up" periodically, and at low energy consumption, to perform a "sniff function" via, for example, a sniff circuit. For the purpose of the present application, the term "sniff function" generally refers to a short, low-power function to determine if a transmitter is present. If a transmitter signal is detected by the sniff function, the device may transition to a higher power communication decode mode. If a transmitter signal is not present, the receiver may return, e.g., immediately return, to sleep mode. In this manner, energy is conserved during relatively long periods when a transmitter signal is not present, while high-power capabilities remain available for efficient decode mode operations during the relatively few periods when a transmit signal is present. Several modes, and combination thereof, may be available for operating the sniff circuit. By matching the needs of a particular system to the sniff circuit configuration, an optimized system may be achieved.

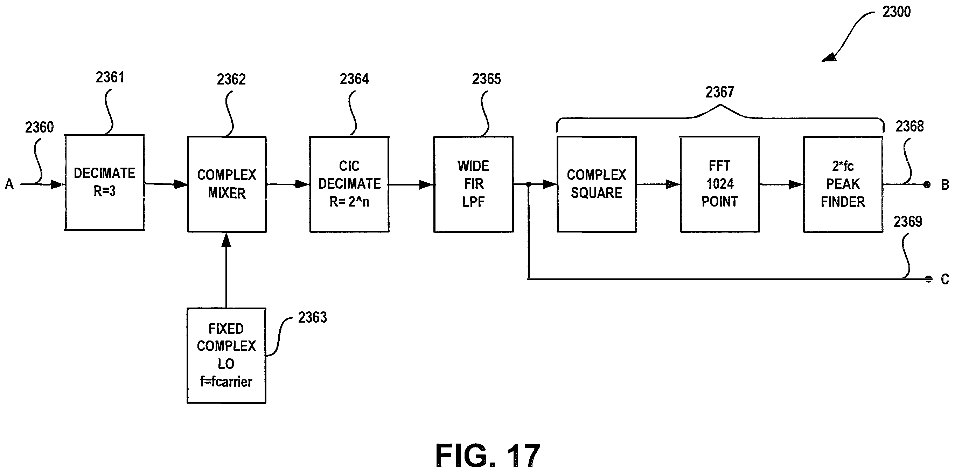

Another view of a beacon module 2300 is provided in the functional block diagram shown in FIG. 17. The scheme outlined in FIG. 17 outlines one technique for identifying a valid beacon. The incoming signal 2360 represents the signals received by electrodes, bandpass filtered (such as from 10 KHz to 34 KHz) by a high frequency signaling chain (which encompasses the carrier frequency), and converted from analog to digital. The signal 2360 is then decimated at block 2361 and mixed at the nominal drive frequency (such as, 12.5 KHz, 20 KHz, etc.) at mixer 2362. The resulting signal is decimated at block 2364 and low-pass filtered (such as 5 KHz BW) at block 2365 to produce the carrier signal mixed down to carrier offsetsignal 2369. Signal 2369 is further processed by blocks 2367 (fast Fourier transform and then detection of two strongest peaks) to provide the true carrier frequency signal 2368. This protocol allows for accurate determination of the carrier frequency of the transmitted beacon.

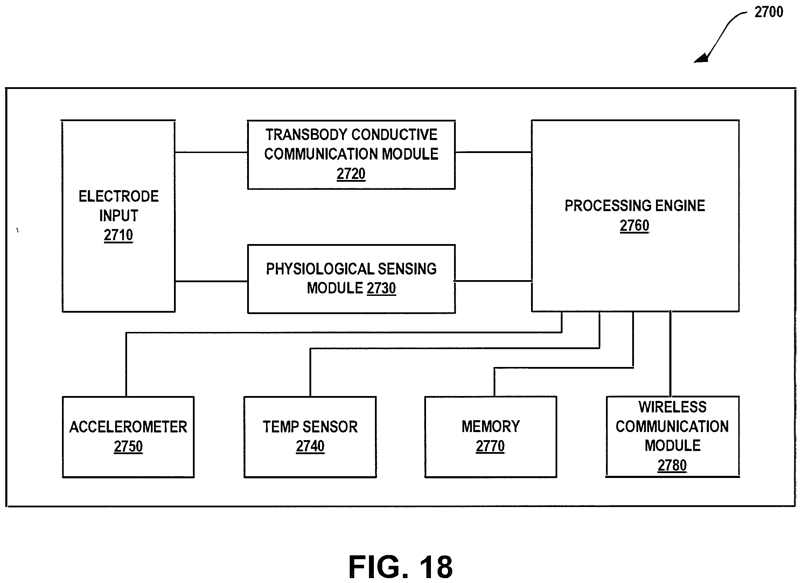

FIG. 18 provides a block functional diagram of an integrated circuit component of a signal receiver according to an aspect of the disclosure. In FIG. 18, a receiver 2700 includes electrode input 2710. Electrically coupled to the electrode input 2710 are transbody conductive communication module 2720 and physiological sensing module 2730. In one aspect, transbody conductive communication module 2720 is implemented as a high frequency (HF) signal chain and physiological sensing module 2730 is implemented as a low frequency (LF) signal chain. Also shown are CMOS temperature sensing module 2740 (for detecting ambient temperature) and a 3-axis accelerometer 2750. Receiver 2700 also includes a processing engine 2760 (for example, a microcontroller and digital signal processor), non-volatile memory 2770 (for data storage) and wireless communication module 2780 (for data transmission to another device, for example in a data upload action).

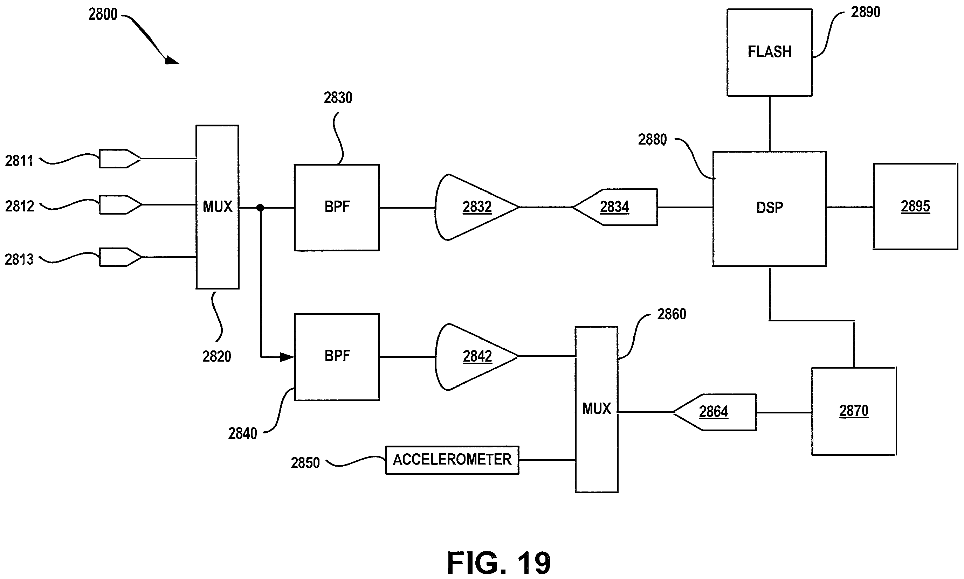

FIG. 19 provides a more detailed block diagram of a circuit configured to implement the block functional diagram of the receiver depicted in FIG. 18, according to one aspect of the disclosure. In FIG. 19, a receiver 2800 includes electrodes e1, e2 and e3 (2811, 2812 and 2813) which, for example, receive the conductively transmitted signals by an IEM and/or sense physiological parameters or biomarkers of interest. The signals received by the electrodes 2811, 2812, and 2813 are multiplexed by multiplexer 2820 which is electrically coupled to the electrodes.

Multiplexer 2820 is electrically coupled to both high band pass filter 2830 and low band pass filter 2840. The high and low frequency signal chains provide for programmable gain to cover the desired level or range. In this specific aspect, high band pass filter 2830 passes frequencies in the 10 KHz to 34 KHz band while filtering out noise from out-of-band frequencies. This high frequency band may vary, and may include, for example, a range of 3 KHz to 300 KHz. The passing frequencies are then amplified by amplifier 2832 before being converted into a digital signal by converter 2834 for input into high power processor 2880 (shown as a DSP) which is electrically coupled to the high frequency signal chain.

Low band pass filter 2840 is shown passing lower frequencies in the range of 0.5 Hz to 150 Hz while filtering out out-of-band frequencies. The frequency band may vary, and may include, for example, frequencies less than 300 Hz, such as less than 200 Hz, including less than 150 Hz. The passing frequency signals are amplified by amplifier 2842. Also shown is accelerometer 2850 electrically coupled to second multiplexer 2860. Multiplexer 2860 multiplexes the signals from the accelerometer with the amplified signals from amplifier 2842. The multiplexed signals are then converted to digital signals by converter 2864 which is also electrically coupled to low power processor (microcontroller) 2870.

In one aspect, a digital accelerometer (such as one manufactured by Analog Devices), may be implemented in place of accelerometer 2850. Various advantages may be achieved by using a digital accelerometer. For example, because the signals the digital accelerometer would produce signals already in digital format, the digital accelerometer could bypass converter 2864 and electrically couple to the low power microcontroller 2870-in which case multiplexer 2860 would no longer be required. Also, the digital signal may be configured to turn itself on when detecting motion, further conserving power. In addition, continuous step counting may be implemented. The digital accelerometer may include a FIFO buffer to help control the flow of data sent to the low power processor 2870. For instance, data may be buffered in the FIFO until full, at which time the processor may be triggered to turn awaken from an idle state and receive the data.

Low power processor 2870 may be, for example, an MSP430 microcontroller from Texas Instruments. Low power processor 2870 of receiver 2800 maintains the idle state, which as stated earlier, requires minimal current draw-e.g., 10µA or less, or 1 µA or less.

High power processor 2880 may be, for example, a VC5509 digital signal process from Texas Instruments. The high power processor 2880 performs the signal processing actions during the active state. These actions, as stated earlier, require larger amounts of current than the idle state-e.g., currents of 30 µA or more, such as 50 µA or more-and may include, for example, actions such as scanning for conductively transmitted signals, processing conductively transmitted signals when received, obtaining and/or processing physiological data, etc.

The receiver may include a hardware accelerator module to process data signals. The hardware accelerator module may be implemented instead of, for example, a DSP. Being a more specialized computation unit, it performs aspects of the signal processing algorithm with fewer transistors (less cost and power) compared to the more general purpose DSP. The blocks of hardware may be used to "accelerate" the performance of important specific function(s). Some architectures for hardware accelerators may be "programmable" via microcode or VLIW assembly. In the course of use, their functions may be accessed by calls to function libraries.

The hardware accelerator (HWA) module comprises an HWA input block to receive an input signal that is to be processed and instructions for processing the input signal; and, an HWA processing block to process the input signal according to the received instructions and to generate a resulting output signal. The resulting output signal may be transmitted as needed by an HWA output block.

Also shown in FIG. 19 is flash memory 2890 electrically coupled to high power processor 2880. In one aspect, flash memory 2890 may be electrically coupled to low power processor 2870, which may provide for better power efficiency.

Wireless communication element 2895 is shown electrically coupled to high power processor 2880 and may include, for example, a BLUETOOTH™ wireless communication transceiver. In one aspect, wireless communication element 2895 is electrically coupled to high power processor 2880. In another aspect, wireless communication element 2895 is electrically coupled to high power processor 2880 and low power processor 2870. Furthermore, wireless communication element 2895 may be implemented to have its own power supply so that it may be turned on and off independently from other components of the receiver-e.g., by a microprocessor.

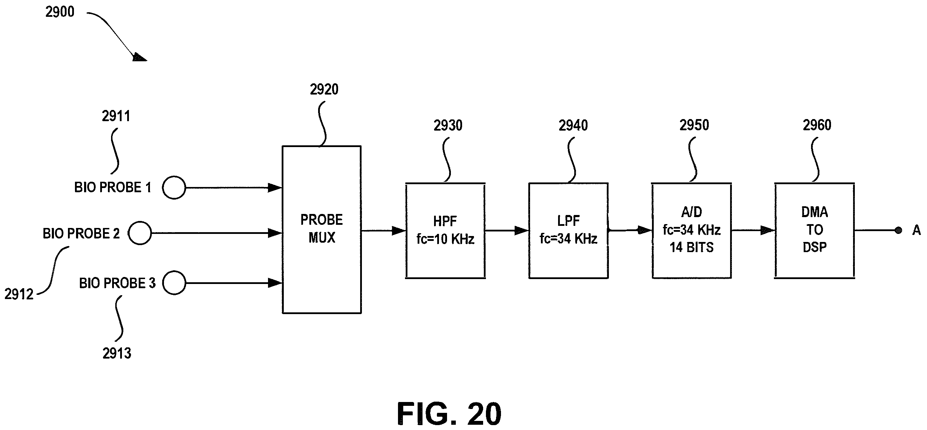

FIG. 20 provides a view of a block diagram of hardware in a receiver according to an aspect of the disclosure related to the high frequency signal chain. In FIG. 20, receiver 2900 includes receiver probes (for example in the form of electrodes 2911, 2912 and 2913) electrically coupled to multiplexer 2920. Also shown are high pass filter 2930 and low pass filter 2940 to provide for a band pass filter which eliminates any out-of-band frequencies. In the aspect shown, a band pass of 10 KHz to 34 KHz is provided to pass carrier signals falling within the frequency band. Example carrier frequencies may include, but are not limited to, 12.5 KHz and 20 KHz. One or more carriers may be present. In addition, receiver 2900 includes analog to digital converter 2950-for example, sampling at 500 KHz. The digital signal can thereafter be processed by the DSP. Shown in this aspect is DMA to DSP unit 2960 which sends the digital signal to dedicated memory for the DSP. The direct memory access provides the benefit of allowing the rest of the DSP to remain in a low power mode.

As stated earlier, for each receiver state, the high power functional block may be cycled between active and inactive states accordingly. Also, for each receiver state, various receiver elements (such as circuit blocks, power domains within processor, etc.) of a receiver may be configured to independently cycle from on and off by the power supply module. Therefore, the receiver may have different configurations for each state to achieve power efficiency.

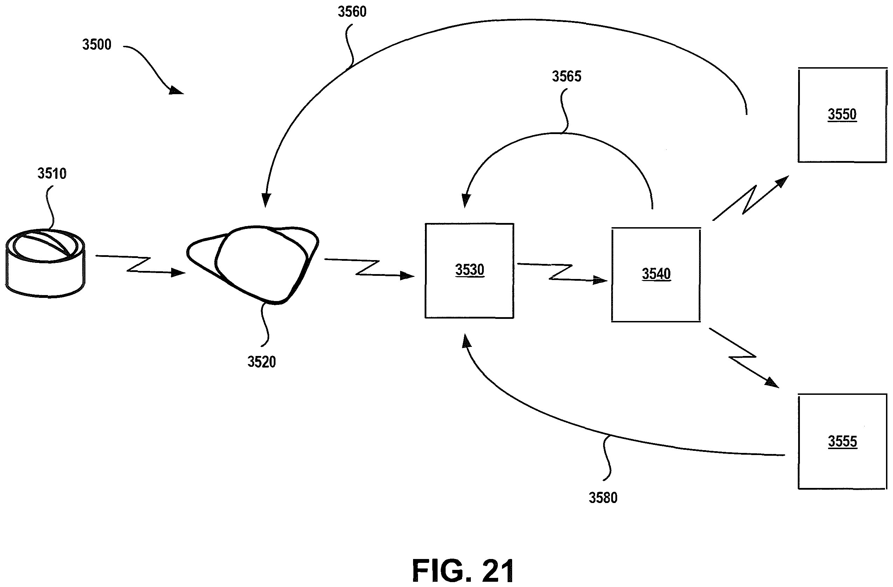

An example of a system of the disclosure is shown in FIG. 21. In FIG. 21, system 3500 includes a pharmaceutical composition 3510 that comprises an IEM. Also present in system 3500 is signal receiver 3520. Signal receiver 3520 is configured to detect a signal emitted from the identifier of the IEM 3510. Signal receiver 3520 also includes physiologic sensing capability, such as ECG and movement sensing capability. Signal receiver 3520 is configured to transmit data to a patient's an external device or PDA 3530 (such as a smart phone or other wireless communication enabled device), which in turn transmits the data to a server 3540. Server 3540 may be configured as desired, e.g., to provide for patient directed permissions. For example, server 3540 may be configured to allow a family caregiver 3550 to participate in the patient's therapeutic regimen, e.g., via an interface (such as a web interface) that allows the family caregiver 3550 to monitor alerts and trends generated by the server 3540, and provide support back to the patient, as indicated by arrow 3560. The server 3540 may also be configured to provide responses directly to the patient, e.g., in the form of patient alerts, patient incentives, etc., as indicated by arrow 3565 which are relayed to the patient via PDA 3530. Server 3540 may also interact with a health care professional (e.g., RN, physician) 3555, which can use data processing algorithms to obtain measures of patient health and compliance, e.g., wellness index summaries, alerts, cross-patient benchmarks, etc., and provide informed clinical communication and support back to the patient, as indicated by arrow 3580.

It is to be understood that this disclosure is not limited to particular aspects described, as such may vary. It is also to be understood that the terminology used herein is for the purpose of describing particular aspects only, and is not intended to be limiting, since the scope of the present disclosure may be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, representative illustrative methods and materials are now described.

Notwithstanding the appended claims, the disclosure is also defined by the following clauses:
1. A composition comprising a shelf-life stability enhancing component and a minimally dimensioned component.
2. The composition according to Clause 1, wherein the composition is an ingestible composition comprising the shelf-life stability component and wherein the minimally dimensioned component can comprise an ingestible component physically associated with the ingestible composition.
3. The composition according to clauses1 or 2 wherein the ingestible composition is stable for 1 year or longer under conditions in which the temperature ranges from 10 to 40°C, the pressure ranges from 0.5 to 2.0 ATM and the relative humidity ranges from 10 to 100%.
4. The composition according to any of the preceding clauses wherein the shelf-life stability enhancing component comprises a water-vapor desensitizer, which preferably comprises a protective barrier that rapidly disrupts upon contact with a liquid.
5. The composition according to Clause 4, wherein the protective barrier comprises a homogeneous layer of a single material or wherein the protective barrier comprises two or more distinct materials.
6. The composition according to Clause 5, wherein the two or more distinct materials are present as a single homogeneous or heterogeneous layer.
7. The composition according to Clause 5 or 6, wherein the two or more distinct materials are present as a multilayer structure.
8. The composition according to any of the clauses 5-7 wherein the two or more distinct materials exhibit different aqueous medium solubility.
9. The composition according to any of the clauses 5-8 wherein the two or more distinct materials exhibit different aqueous medium physical properties.
10. The composition according to any of the clauses 5-9 wherein the two or more distinct materials comprise a first material and a second material that solubilizes the first material.
11. The composition according to any of the preceding clauses wherein the shelf-life stability enhancing component comprises a lipid, or functionally analogous material.
12. The composition according to any of the preceding clauses wherein the shelf-life stability enhancing component comprises a low-melting point material.
13. The composition to any of the preceding clauses wherein the shelf-life stability enhancing component comprises a galvanic protective barrier.
14. The composition according to any of the preceding clauses wherein the shelf-life stability enhancing component is configured to be disruptable from within when ingested.
15. The composition according to any of the preceding clauses wherein the shelf-life stability enhancing component is configured to provide aqueous liquid passage there through upon contact of the ingestible composition with an aqueous liquid.
16. The composition according to any of the preceding clauses 4-15 wherein the water-vapor desensitizer comprises a desiccant.
17. The ingestible composition according to any of the preceding clauses wherein the minimally dimensioned component composition comprises one or more of the following:
   an ingestible device
   a micro battery
   a pharmaceutically active agent,
   a diagnostic agent.
18. The ingestible composition according to Clause 17, wherein the ingestible device comprises a mechanical and/or electrical component, for example where the device comprises a circuitry component and wherein preferably the device comprise an ingestible event marker (IEM).
19. The ingestible composition according to Clause 18 wherein the IEM comprises a control device for altering conductance and a power source, preferably wherein the power source is a partial power source which includes a first material electrically coupled to the control device and a second material electrically coupled to the control device and electrically isolated from the first material.
20. The ingestible composition according to clause 19 wherein the ingestible event marker is configured to form a current path, when in contact with a conducting fluid, preferably a bodily conducting fluid, between the first material and the second material.
21. The ingestible composition according to clause 20 wherein a voltage potential created between the first and second materials- when the ingestible composition is ingested-provides power for operating the IEM.
22. The ingestible composition according to any of the clauses 18-21 wherein the IEM further comprises a current path extender, preferably in the form of a membrane.
23. A system comprising an ingestible composition according to any of the preceding clauses and a receiver configured to receive a communication associated with the ingestible composition.
24. A process for providing an ingestible composition according to any of the preceding clauses 1-22 comprising combining a minimally dimensioned component and a shelf-life stability enhancing component.
25. Use of a system according to clause 23 for providing information.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present disclosure is not entitled to antedate such publication by virtue of prior disclosure. Further, the dates of publication provided may be different from the actual publication dates which may need to be independently confirmed.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As may be apparent to those of skill in the art upon reading this disclosure, each of the individual aspects described and illustrated herein has discrete components and features which may be readily separated from or combined with the features of any of the other several aspects without departing from the scope or spirit of the present disclosure. Any recited method can be carried out in the order of events recited or in any other order which is logically possible.

Although the foregoing disclosure has been described in some detail by way of illustration and example for purposes of clarity of understanding, it is readily apparent to those of ordinary skill in the art in light of the teachings of this disclosure that certain changes and modifications may be made thereto without departing from the spirit or scope of the appended claims.

Accordingly, the preceding merely illustrates the principles of the disclosure. It may be appreciated that those skilled in the art may be able to devise various arrangements which, although not explicitly described or shown herein, embody the principles of the disclosure and are included within its spirit and scope. Furthermore, all examples and conditional language recited herein are principally intended to aid the reader in understanding the principles of the disclosure and the concepts contributed by the inventors to furthering the art, and are to be construed as being without limitation to such specifically recited examples and conditions. Moreover, all statements herein reciting principles, aspects, and aspects of the disclosure as well as specific examples thereof, are intended to encompass both structural and functional equivalents thereof. Additionally, it is intended that such equivalents include both currently known equivalents and equivalents developed in the future, e.g., any elements developed that perform the same function, regardless of structure. The scope of the present disclosure, therefore, is not intended to be limited to the exemplary aspects shown and described herein. Rather, the scope and spirit of present disclosure is embodied by the appended claims.

The invention is further defined in the following clauses:
Clause 1: A composition comprising a shelf-life stability component physically associated with a minimally dimensioned component.
Clause 2: The composition according to clause 1, wherein the composition is an ingestible composition comprising the shelf-life stability component and an ingestible component physically associated with the ingestible composition.
Clause 3: The ingestible composition according to clause 2, wherein the composition further comprises an ingestible device.
Clause 4: The ingestible composition according to clause 3, wherein the ingestible device is an ingestible event marker.
Clause 5: The composition according to clause 1, wherein the ingestible composition is stable for 1 year or longer under conditions in which the temperature ranges from 10 to 40°C, the pressure ranges from 0.5 to 2.0 ATM and the relative humidity ranges from 10 to 100%.
Clause 6: The composition according to clause 1, wherein the shelf-life stability component comprises a water-vapor desensitizer.
Clause 7: The composition according to clause 6, wherein the water-vapor desensitizer comprises a protective barrier that rapidly disrupts upon contact with a liquid.
Clause 8: The composition according to clause 7, wherein the protective barrier comprises a homogeneous layer of a single material.
Clause 9: The composition according to clause 7, wherein the protective barrier comprises two or more distinct materials.
Clause 10: The composition according to clause 9, wherein the two or more distinct materials are present as a single homogeneous or heterogeneous layer.
Clause 11: The composition according to clause 9, wherein the two or more distinct materials are present as a multilayer structure.
Clause 12: The composition according to clause 9, wherein the two or more distinct materials exhibit different aqueous medium solubility.
Clause 13: The composition according to clause 9, wherein the two or more distinct materials exhibit different aqueous medium physical properties.
Clause 14: The composition according to clause 9, wherein the two or more distinct materials comprise a first material and a second material that solubilizes the first material.
Clause 15: The composition according to clause 7, wherein the protective barrier comprises a lipid.
Clause 16: The composition according to clause 7, wherein the protective barrier comprises a low-melting point material.
Clause 17: The composition according to clause 7, wherein the protective barrier is a galvanic protective barrier.
Clause 18: The composition according to clause 7, wherein the protective barrier is configured to be disruptable by a device present in the composition.
Clause 19: The composition according to clause 7, wherein the protective barrier is configured to provide aqueous liquid passage through the protective barrier upon contact of ingestible composition with an aqueous liquid.
Clause 20: The composition according to clause 7, wherein the protective barrier comprises a liquid passageway.
Clause 21: The composition according to clause 6, wherein the water-vapor desensitizer comprises a desiccant.
Clause 22: The composition according to clause 1, wherein minimally dimensioned component is a micro-battery.
Clause 23: A system comprising:
   an ingestible composition comprising:
   a shelf-life stability component; and
   an ingestible component associated with the shelf-life stability component; and
   a receiver configured to receive a communication associated with the ingestible composition.
Clause 24: A method comprising combining a minimally dimensioned component and a shelf-life stability component.
Clause 25: The method according to clause 24, wherein the minimally dimensioned component is an ingestible component and the method produces an ingestible composition.
Clause 26: The method according to clause 25, wherein the method comprises stably associating the ingestible component and the shelf-life stability component.
Clause 27: The method according to clause 26, wherein the method comprises one or more protocols selected from the group consisting of laminating, pressing, stamping, extruding, molding and coating.
Clause 28: The method according to clause 27, wherein at least a portion of the method is automated.

## Claims

1. A composition comprising:
an ingestible component comprising an ingestible event marker and a pharmaceutically acceptable carrier; and
a shelf-life stability component enclosing the ingestible event marker to provide a protective barrier for the ingestible event marker, wherein at least a portion of the shelf-life stability component is configured to disrupt when the shelf-life stability component is in contact with a liquid;
wherein the ingestible event marker comprises:
a control device; and
a partial power source comprising first and second electrodes formed of dissimilar electrochemical materials configured to contact an electrically conductive fluid and generate a voltage to energize the ingestible event marker; and
wherein the energized ingestible event marker is configured to produce a conductive current flow through the electrically conductive fluid; and
wherein the control device is configured to control the conductive current flow;
wherein the shelf-life stability component comprises a water-vapor desensitizer comprising a desiccant;
wherein the desiccant comprises an indicator compound that provides an indication of whether or not the desiccant has reached capacity with respect to an amount of water the desiccant is able to sequester;
wherein the shelf-life stability component comprises a first material and a second material adjacent the first material; and
wherein the first material and the second material exhibit different aqueous medium solubility.

2. The composition according to claim 1, which is stable for more than 1 years under conditions of a temperature in the range of 10 to 40 ° C., a pressure in the range of 0.5 to 2.0 atmospheres, and a relative humidity in the range of 10 to 100%.

3. A composition comprising:
an ingestible component comprising an ingestible event marker and a pharmaceutically acceptable carrier; and
a shelf-life stability component enclosing the ingestible event marker to provide a protective barrier for the ingestible event marker, wherein at least a portion of the shelf-life stability component is configured to disrupt when the shelf-life stability component is in contact with a liquid;
wherein the ingestible event marker comprises:
a control device; and
a partial power source comprising first and second electrodes formed of dissimilar electrochemical materials configured to contact an electrically conductive fluid and generate a voltage to energize the ingestible event marker;
wherein the energized ingestible event marker is configured to produce a conductive current flow through the electrically conductive fluid;
wherein the control device is configured to control the conductive current flow;
wherein the shelf-life stability component comprises a water-vapor desensitizer comprising a desiccant;
wherein the desiccant comprises an indicator compound that provides an indication of whether or not the desiccant has reached capacity with respect to an amount of water the desiccant is able to sequester; and
wherein the protective barrier comprises a homogeneous sheet of a single material in contact with the first and second electrodes of the ingestible event marker.

4. The composition according to claim 1, wherein the first material and the second material are present as a single homogeneous layer or a heterogeneous layer.

5. The composition of claim 1, wherein the first material and the second material exist as a multilayer structure.

6. The composition according to claim 1, wherein the first material comprises a first layer and a second layer covering the first layer, and the second material separates the first layer and the second layer of the first material.

7. The composition of claim 1, wherein said first material is at least in contact with said first electrode and said first material is more soluble than said second material.

8. The composition according to claim 1, wherein said second material solubilizes said first material.

9. The composition of claim 1, wherein the storage stability element comprises a lipid.

10. The composition of claim 1, wherein the storage stability element comprises a low melting point material.

11. The composition of claim 1, wherein the storage stability element comprises a galvanic protective barrier.

12. The composition of claim 1, wherein the protective barrier is configured to be destroyed by the ingestible event marker.

13. The composition of claim 1, wherein the composition is configured to form an aqueous liquid passage through the element.

14. The composition of claim 1, wherein the shelf-life stability component includes a liquid passage, and wherein the composition is filled with a material that dissolves upon contact with an aqueous liquid medium and is configured to provide access to at least one of the first electrode and the second electrode of the ingestible event marker through the liquid passage to the aqueous liquid medium.

15. A composition comprising:
an ingestible component comprising an ingestible event marker and a pharmaceutically acceptable carrier; and
a shelf-life stability component entirely enclosing the ingestible event marker to provide a protective barrier for the ingestible event marker, wherein at least a portion of the shelf-life stability component is configured to disrupt when the shelf-life stability component is in contact with a liquid;
wherein the ingestible event marker comprises:
a control device; and
a partial power source comprising first and second electrodes formed of dissimilar electrochemical materials configured to contact an electrically conductive fluid and generate a voltage to energize the ingestible event marker;
wherein the energized ingestible event marker is configured to produce a conductive current flow through the electrically conductive fluid;
wherein the control device is configured to control the conductive current flow;
wherein the shelf-life stability component comprises a water-vapor desensitizer comprising a desiccant;
wherein the desiccant comprises an indicator compound that provides an indication of whether or not the desiccant has reached capacity with respect to an amount of water the desiccant is able to sequester; and
wherein the protective barrier comprises a liquid passageway; and
wherein the liquid passageway is filled with a material that is configured to dissolve upon contact with an aqueous liquid medium and provide the aqueous liquid medium the access to at least one of the first electrode or the second electrode of the ingestible event marker through the liquid passageway.
